(19) Europäisches Patentamt
European Patent Office
Office européen des brevets

(11) EP 1 726 294 B1

(12) FASCICULE DE BREVET EUROPEEN

(45) Date de publication et mention de la délivrance du brevet:
**09.06.2010 Bulletin 2010/23**

(21) Numéro de dépôt: **06113857.4**

(22) Date de dépôt: **12.05.2006**

(51) Int Cl.:
***A61K 8/36*** *(2006.01)* ***A61K 8/44*** *(2006.01)*
***A61K 8/45*** *(2006.01)* ***A61K 8/46*** *(2006.01)*
***A61K 8/898*** *(2006.01)* ***A61Q 5/02*** *(2006.01)*
***A61Q 5/12*** *(2006.01)* ***A61K 8/73*** *(2006.01)*
***A61Q 1/14*** *(2006.01)*

(54) **Compositions cosmétiques détergentes comprenant une silicone aminée pipéridine et un polymère cationique et utilisation de ces dernières**

Kosmetische Waschmittelzusammensetzungen enthaltend ein Amino Piperidin-Silikon und ein kationisches Polymer und ihre Verwendung

Detergent cosmetic compositions comprising an amino piperidine silicone and a cationic polymer and uses thereof

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.05.2005 FR 0551351**

(43) Date de publication de la demande:
**29.11.2006 Bulletin 2006/48**

(73) Titulaire: **L'Oréal**
**75008 Paris (FR)**

(72) Inventeur: **Maubru, Mireille**
**78400 Chatou (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 0 739 625** **EP-A- 0 974 335**
**WO-A-97/46211** **US-A1- 2003 134 760**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Printed by Jouve, 75001 PARIS (FR)

EP 1 726 294 B1

## Description

La présente invention concerne de nouvelles compositions cosmétiques à propriétés améliorées destinées simultanément au nettoyage, au conditionnement des matières kératiniques notamment des cheveux, et comprenant, dans un support cosmétiquement acceptable, une base lavante constituée de tensio-actifs à pouvoir détergent dans laquelle sont également présents des silicones aminées particulières en association avec des polymères cationiques. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

De plus, les compositions notamment limpides contenant des silicones aminées ont une coloration qui évolue dans le temps.

Enfin, les shampoings peuvent entraîner un phénomène de dégorgement de la coloration cosmétique des cheveux non négligeable. La couleur des cheveux s'affaiblit peu à peu ou vire vers des couleurs peu esthétiques ou non souhaitables.

US2003/130760 décrit des compositions de shampooings comprenant une silicone à amine encombrée. EP 0 739 625 divulgue des compositions détergentes comprenant un tensioactif anionique, un tensioactif amphotère et un céramide. EP 0 974 335 et WO97/46211 divulguent des compositions détergentes comprenant un tensioactif anionique, un tensioactif amphotère et une silicone aminée.

Le but de la présente invention est d'obtenir des compositions détergentes suffisamment moussantes ayant de bonnes propriétés cosmétiques et ne dégradant pas ou peu la couleur (coloration) synthétique (artificielle) des cheveux. De plus la couleur des compositions ne doit pas ou peu évoluer dans le temps.

Ainsi, à la suite d'importantes recherches menées sur la question, il a maintenant été trouvé par la Demanderesse, de façon totalement inattendue et surprenante, qu'en associant des silicones aminées, tels que définis ci-après, avec des polymères cationiques dans des compositions détergentes, il est possible d'améliorer de manière substantielle et significative les propriétés de lissage visuel et au toucher des cheveux, et ceci tout en ayant un bon pouvoir lavant et moussant et en conservant leurs autres propriétés cosmétiques.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse sans aucun effet de charge.

Par ailleurs, la couleur des compositions selon l'invention n'évolue pratiquement pas au cours du temps.

Enfin, les compositions selon l'invention ont un effet peu dégradant vis-à-vis de la coloration synthétique (artificielle) des cheveux.

Ainsi, selon la présente invention, il est maintenant proposé de nouvelles compositions de lavage des matières kératiniques, en particulier des cheveux comprenant, dans un milieu aqueux cosmétiquement acceptable, au moins un tensioactif détergent anionique, non ionique ou amphotère, au moins un polymère cationique et au moins une silicone aminée particulière, définie ci-après, comprenant une fonction amine portée par un groupe stériquement encombré.

L'invention a également pour objet l'utilisation en cosmétique des compositions ci-dessus pour le nettoyage, le conditionnement, le soin des matières kératiniques notamment les cheveux et les cils.

Un autre objet de l'invention consiste en un procédé de traitement cosmétique des matières kératiniques notamment des fibres kératiniques mettant en oeuvre la composition selon l'invention et plus particulièrement les cheveux et les cils.

L'invention a encore pour objet une utilisation de la composition selon l'invention comme shampoing.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.
Le ou les tensioactifs détergents sont choisis, seuls ou en mélanges, au sein des tensioactifs anioniques, amphotères, non ioniques et zwittérioniques.
Ainsi, selon l'invention, les tensioactifs détergents peuvent représenter de 4 % à 50 % en poids, de préférence de 6 % à 35 % en poids, et encore plus préférentiellement de 8 % à 25 % en poids, du poids total de la composition finale.
Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique.
Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\text{-}CONHCH_2CH_2\text{-}N^+(R_3)(R_4)(CH_2COO^-) \quad (2)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-}CONHCH_2CH_2\text{-}N(B)(C) \qquad (3)$$

dans laquelle :

B représente $-CH_2CH_2OX'$, C représente $-(CH_2)_z-Y'$, avec z = 1 ou 2,
X' désigne le groupement $-CH_2CH_2-COOH$ ou un atome d'hydrogène
Y' désigne -COOH ou le radical $-CH_2 - CHOH - SO_3H$
$R_2'$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

A titre d'exemple on peut citer le cocoamphocarboxyglycinate vendu sous la dénomination commerciale MIRANOL C2M concentré par la Société MIRANOL.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et au moins un agent tensioactif amphotère.

La quantité d'agents tensioactifs anioniques va de préférence de 3 % à 40% en poids, rapportée au poids total de la composition cosmétique. Elle est de préférence comprise entre 5 % et 35 % en poids et, mieux encore, entre 8 % et 25 % en poids.

La quantité d'agents tensioactifs amphotères et/ou non ioniques, lorsqu'ils sont présents, va de préférence comprise de 0,5 à 20 % en poids, et en particulier de 1 à 15 % en poids et plus particulièrement de 2 à 10 % en poids rapportée au poids total de la composition.

<u>Silicone aminée stériquement encombrée</u>

Par silicone aminée comportant une fonction amine portée par un groupement stériquement encombré, on entend au sens de la présente invention toute silicone possédant à l'une au moins de ses extrémités et/ou sur des chaînes pendantes au moins un groupement aminé intégré dans un hétérocycle composé de 5 à 8 chaînons, les carbone en $\alpha$, et $\alpha'$ de l'atome d'azote étant totalement substitués par des atomes ou groupements différents de l'hydrogène.

Les silicones aminées utilisées dans l'invention comprenant une fonction amine portée par un groupe stériquement encombré sont les polyorganosiloxanes ayant par mole au moins un motif de formule générale (I) :

$$\text{(I)} \qquad (R)_a(X)_bZSi(O)_{\frac{3-(a+b)}{2}}$$

dans laquelle :

- les symboles R sont identiques ou différents et représentent un radical hydrocarboné monovalent choisi parmi les radicaux alkyles, linéaires ou ramifiés, ayant de 1 à 4 atomes de carbone, phényle, benzyle et trifluro-3,3,3 propyle;
- les symboles X sont identiques ou différents et représentent un radical monovalent choisi parmi un groupement hydroxyle et un radical alkoxy, linéaire ou ramifié, ayant de 1 à 3 atomes de carbone ;
- Z représente un radical monovalent comprenant une fonction amine portée par un groupe stériquement encombré, à savoir un ou plusieurs groupe(s) pipéridinyle(s) stériquement encombrés choisi parmi :

1) les radicaux de formule (II) :

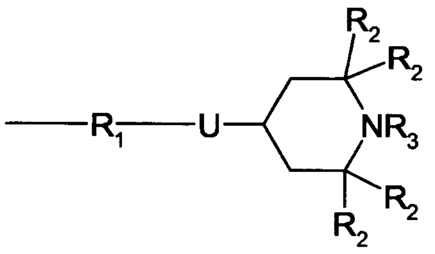 (II)

dans laquelle :

A) $R_1$ est un radical hydrocarboné divalent comprenant éventuellement un ou plusieurs hétéroatomes tels que O et N choisi parmi :

a) Les radicaux alkylènes, linéaires ou ramifiés, ayant de 2 à 18 atomes de carbone ;
b) Les radicaux alkylène-carbonyle dont la partie alkylène, linéaire ou ramifiée, comporte 2 à 20 atomes de carbone ;
c) Les radicaux alkylène-cyclohexylène dont la partie alkylène, linéaire ou ramifiée, comporte de 1 à 12 atomes de carbone et la partie cyclohexylène comporte un groupement OH et éventuellement 1 ou 2 radicaux alkyles ayant de 1 à 4 atomes de carbone ;
d)Les radicaux de formule $—R_4\text{-O-}R_5—$ dans laquelle les radicaux R4 et R5 identiques ou différents représentent des radicaux alkylènes ayant 1 à 12 atomes de carbone;
e)Les radicaux de formule $—R_4\text{-O-}R_5—$ dans laquelle les radicaux $R_4$ et $R_5$ ont les significations indiquées précédemment l'un d'entre eux ou les deux sont substitués par un ou deux groupement(s) OH ;
f) Les radicaux de formules $—R_4\text{-COO-}R_5—$ et $\text{-}R_4\text{-OCO-}R_5—$ dans lesquelles $R_4$ et $R_5$ ont les significations précédentes ;
g)Les radicaux de formule $\text{-}R_6\text{-O-}R_7\text{-O-CO-}R_8\text{-}$ dans laquelle $R_6$, $R_7$ et $R_8$, identiques ou différents, représentent des radicaux alkylènes ayant de 2 à 12 atomes de carbone et le radical $R_7$ est éventuellement substitué par un groupement hydroxyle ;

B) U représente -O- ou -NRg-, $R_9$ étant un radical monovalent choisi parmi un atome d'hydrogène un radical alkyle, linéaire ou ramifié, ayant de 1 à 6 atomes de carbone; un radical divalent $\text{-}R_1\text{-}$ ayant la signification indiquée précédemment, l'un des liens valentiels étant relié à l'atome d'azote de $\text{-NR}_9\text{-}$, l'autre étant relié à un atome de silicium ;

C)$R_2$ sont des radicaux, identiques ou différents, choisis parmi les radicaux alkyles, linéaires ou ramifiés, ayant de 1 à 3 atomes de carbone et phényle ;

D) $R_3$ représente un atome d'hydrogène ou le radical $R_2$ ;

- a est un nombre choisi parmi 0, 1 et 2 ;
- b est un nombre choisi parmi 0, 1, et 2 ;
- la somme a + b est au plus égale à 2.

Le polyorganosiloxane utilisé peut comprendre en outre au moins un autre motif siloxyle de formule :

$$\text{(III)} \qquad (R)_c(X)_d VSi(O)_{\frac{3-(c+d)}{2}}$$

dans laquelle :

- les symboles R et X ont les mêmes significations que celles données ci avant à propos de la formule (I) ;

- le symbole V représente: un radical alkyle, linéaire ou ramifié, ayant de 5 à 20 atomes de carbone; un radical de formule -$(CH_2)$p-$COOR_{12}$ dans laquelle p représente un nombre de 5 à 20 et $R_{12}$ représente un radical alkyle linéaire

ou ramifié de 1 à 12 atomes de carbone ; un radical de formule

- $(CH_2)_q$-O- $R_{13}$ dans laquelle q représente un nombre de 3 à 10 et $R_{13}$ représente un atome d'hydrogène, un oxyde d'éthylène, un groupement mono

ou polyoxyéthyléné, un groupement mono ou polyoxypropyléné, un enchaînement mixte constitué de motifs d'oxyde d'éthylène et d'oxyde de propylène ou un radical acyle ayant de 2 à 12 atomes de carbone ;

- c est un nombre choisi parmi 0, 1 et 2 ;
- d est un nombre choisi parmi 0, 1 et 2 ;
- la somme c + d est au plus égale à 2.

Le polyorganosiloxane mis en oeuvre peut comprendre en outre d'autre(s) motif(s) siloxyle(s) de formule (IV):

$$\text{(IV)} \quad (R)_e(X)_f Si(O)_{\frac{4-(e+f)}{2}}$$

dans laquelle :

- R et X ont les mêmes significations que celles données à propos de la formule (I) ;
- e est un nombre choisi parmi 0, 1, 2 et 3 ;
- f est un nombre choisi parmi 0, 1, 2 et 3 ;
- La somme e + f est au plus égale à 3.

Les motifs siloxyles de formule (I), quand il y en a plus de deux, peuvent être identiques ou différents entre eux; la même remarque s'applique également aux motifs siloxyles de formules (III) et (IV).
Les différents motifs sont de préférence répartis statistiquement le long de la chaîne.
Le polyorganosiloxane mis en oeuvre peut présenter une structure linéaire cyclique ou ramifiée (présence de motifs de types T et/ou Q) ou mélange de ces structures. Dans le cas de la présence de motifs monoorganosiloxy de type T et/ou de motifs Q ($SiO_2$), ces motifs sont dans la proportion d'au plus 10% par rapport au nombre de motifs diorganosiloxy de type D.
Le polyorganosiloxane mis en oeuvre est, par exemple, un polydiorganosiloxane linéaire de formule moyenne (V) :

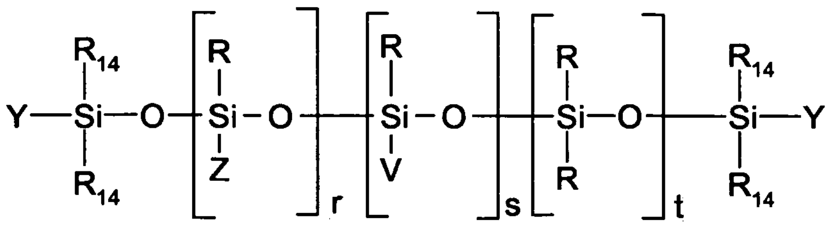

(V)

Dans laquelle :

- Les différents motifs sont de préférence répartis statistiquement le long de la chaîne.

- Les symboles R, Z et V ont les significations données ci avant à propos des formules (I) et (III) ;

- Le symbole Y représente un radical monovalent choisi parmi les radicaux R, Z, V et X;

- Les symboles $R_{14}$ sont identiques ou différents et représentent un radical monovalent choisi parmi un radical R et un radical X tel que défini ci avant à propos de la formule (I) ;

- r, s et t sont égaux à zéro ou représentent des nombres entiers ou fractionnaires supérieurs à zéro, avec la condition supplémentaire selon laquelle si r = 0, au moins un des deux radicaux Y représente le radical Z.

De préférence, la composition selon l'invention comprend un polyorganosiloxane à motifs(s) de formules (I) + éventuellement (III) + éventuellement (IV) ou un polyorganosiloxane de formule (V) :
Ces polyorganosiloxanes ont en moyenne par mole : de 2 à 1600 atomes de silicium, de 1 à 100 radicaux Z tels que ceux ci-après définis et de 0 à 50 radicaux V tels que ceux ci-après définis ;

- et dans la structure duquel :

  - R est choisi parmi les radicaux méthyle, éthyle, n-propyle et isopropyle, de préférence méthyle ;
  - X est choisi parmi les radicaux hydroxyle, méthoxy et éthoxy ;
  - Z est choisi parmi les radicaux à groupes(s) pipéridinyle(s) :

    1) de formule (II) dans laquelle :

    A) $R_1$ est un radical hydrocarboné divalent comprenant éventuellement un ou plusieurs hétéroatomes tels que O et N choisi parmi :

    a) Les radicaux alkylènes, linéaires ou ramifiés, ayant de 2 à 18 atomes de carbone ;
    b) Les radicaux alkylènes, linéaires ou ramifiés, ayant de 3 à 12 atomes de carbone ;
    c) Le radical $—(CH_2)_{10}$-CO-;
    d) Les radicaux alkynèle-cyclohexylène dont la partie alkylène, linéaire ou ramifiée, comporte de 2 à 6 atomes de carbone et la partie cyclohylène comporte un groupement -OH et éventuellement 1 ou 2 subsistants alkyles ayant 1 à 4 atomes de carbone ;
    e) Les radicaux de formule $-R_4-O-R_5-$ dans laquelle les radicaux $R_4$ et $R_5$ identiques ou différents représentent des radicaux alkylènes ayant 2 à 6 atomes de carbone ;
    f) Les radicaux de formule $-R_4-O-R_5-$ dans laquelle les radicaux $R_4$ et $R_5$ ont les significations indiquées précédemment et R5 est substitué par un groupe OH ;
    g) Les radicaux de formules $-R_4-COO-R_5-$ et $-R_4-OCO-R_5-$ dans lesquelles $R_4$ et $R_5$ ont les significations précédentes ;
    h) Les radicaux de formule $-R_6-O-R_7-O-CO-R_8-$ dans laquelle $R_6$, $R_7$, $R_8$, identiques ou différents, représentent des radicaux alkylènes ayant de 2 à 6 atomes de carbone et le radical $R_7$ est substitué par un groupement hydroxyle ;

    B) U représente -O- ou $-NR_9-$ où $R_9$ est un radical alkyle, linéaire ou ramifié, ayant de 1 à 4 atomes de carbone;
    C) $R_2$ représente un radical méthyle;

  - V est choisi parmi: un radical alkyle, linéaire ou ramifié, ayant 5 à 18 atomes de carbone; un radical de formule $-(CH_2)_{10}-COO-R_{12}$ laquelle $R_{12}$ représente un radical alkyle, linéaire de 1 à 6 atomes de carbone, un radical de formule $-(CH_2)_3-O-R_{13}$, où $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un groupement acyle ayant de 2 à 6 atomes de carbone.

Dans le cadre de cette modalité préférentielle, le polydiorganosiloxane linéaire de formule (V) présente les valeurs suivantes pour les symboles r, s et t :

- r est un nombre entier ou fractionnaire allant de 0 à 98 avec la condition selon laquelle si r = 0, au moins un des deux radicaux Y représente le radical Z

- s est un nombre entier ou fractionnaire allant de 0 à 48 ;

- t est un nombre entier ou fractionnaire allant de 0 à 1598 ;

- la somme r + s + t est un nombre entier ou fractionnaire allant de 0 à 1598.

De manière plus préférentielle, la composition selon l'invention comprend un polyorganosiloxane à motifs de formule (I) + (IV) + éventuellement (III) ou un polyorganosiloxane de formule (V) :

- qui a en moyenne par mole : de 5 à 800 atomes de silicium, de 1 à 60 radicaux Z tels que ceux ci-après définis et de 0 à 20 radicaux V tels que ceux ci-après définis ;

  - et dans la structure duquel :

    - R est un radical méthyle ;
    - X est choisi parmi les radicaux hydroxyle et méthyle ;
    - Z est choisi parmi les radicaux à groupe(s) pipéridinyle(s) :

      ➢ De formule (II) dans laquelle :

      - $R_1$ représente un radical: triméthylène ; décaméthylène-carbonyle ; hydroxy-2 oxa-4 heptaméthylène ; hydroxy-6 dioxa-4,8 oxo-3 undécaméthylène, de préférence triméthylène ;
        U représente -O- ou $NR_9$ où $R_9$ est choisi parmi les radicaux méthyle, éthyle, n-propyle et n-butyle ;

      - $R_2$ représente un radical méthyle;

      - $R_3$ représente un atome d'hydrogène;

    - V est choisi parmi les radicaux n-octyle et décaméthylène carboxylate de méthyle ou d'éthyle
      Dans le cadre de cette modalité plus préférentielle, le polydiorganosiloxane linéaire de formule (V) présente les valeurs suivantes pour les symboles r, sett:
    - r est un nombre entier ou fractionnaire allant de 0 à 58 avec la condition selon laquelle si r = 0, au moins un des deux radicaux Y représente le radical Z ;
    - s est un nombre entier ou fractionnaire allant de 0 à 18 ;
    - t est un nombre entier ou fractionnaire allant de 3 à 798 ;
    - La somme r + s + t est un nombre entier ou fractionnaire allant de 3 à 798.

Généralement, 100 g de polyorganosiloxane tel que ceux précédemment définis comprennent 1 à 200 milliéquivalents (meq) de groupe(s) pipéridinyle(s) encombrés(s), de préférence de 2 à 100 et plus particulièrement de 5 à 50 meq.
Le pourcentage d'azote de la silicone aminée va de préférence de 0,05% à 1% en poids par rapport au poids total de la silicone, de préférence de 0,1 à 0,5% en poids.
Les silicones préférées selon l'invention présentent notamment la structure suivante :

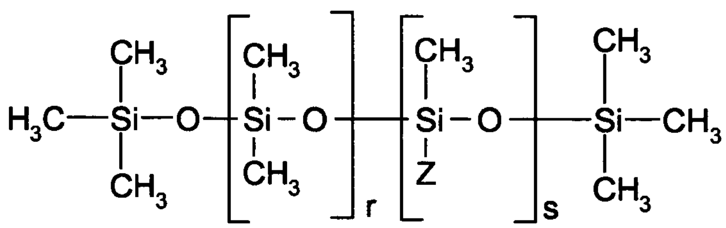

avec Z =

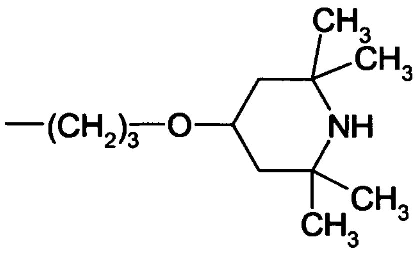

s varie de 1 à 50, de préférence de 2 à 30
r varie de 50 à 2000, de préférence de 500 à 1700.

De telles silicones aminées comprenant un groupe fonctionnel stériquement encombré sont par exemple proposées par la société RHODIA sous la dénomination RHODORSIL® Huile 21645, RHODORSIL® Huile 21650, JLR 3442 et JLR 3440.

Les silicones aminées à groupements stériquement encombrés peuvent se présenter sous forme de solutions, de dispersions, de microémulsions ou d'émulsions.

Selon l'invention, la ou les silicones aminées selon l'invention peuvent représenter de 0,001 % à 10 % en poids, de préférence de 0,01 % à 5% en poids et plus particulièrement de 0,1 à 3% en poids par rapport au poids total de la composition finale.

Selon l'invention, les compositions comprennent en outre au moins un polymère cationique.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863 et ayant une densité de charge cationique convenable.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre ou en poids comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

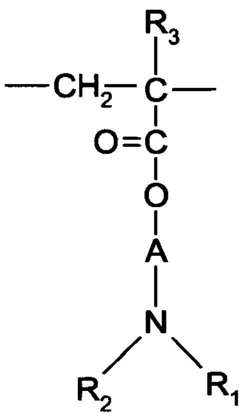
,
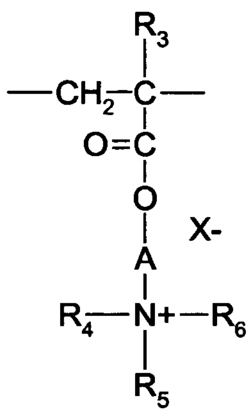

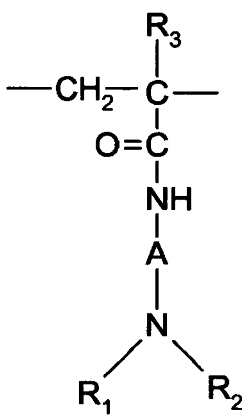

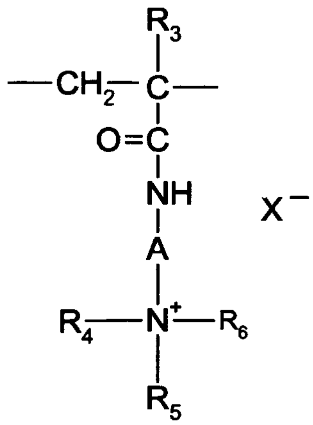

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.
Les copolymères de la famille (1) peuvent contenir en outre un ou plusieurs motifs dérivant de monomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymères d'acrylamide et de méthosulfate de méthacryloyloxyéthyltriméthylammonium vendus sous la dénomination RETEN par la société HERCULES,
- les copolymères vinylpyrrolidone / acrylate ou méthacrylate de dialkylaminoalkyle quaternisés ou non. Ces polymères sont décrits en détail dans les brevets français 2.077.143 et 2.393.573,
- les terpolymères méthacrylate de diméthyl amino éthyle/vinylcaprolactame/ vinylpyrrolidone,
- les copolymères vinylpyrrolidone / méthacrylamidopropyl dimethylamine.
- et les copolymères vinylpyrrolidone / méthacrylamide de diméthylaminopropyle quaternisé.

(2) Les polysaccharides cationiques notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
Les dérivés d'éthers de cellulose comportant des groupements ammonium quaternaires décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
Les copolymères de cellulose cationiques ou les dérivés de cellulose greffés avec un monomère hydrosoluble

d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
Les gommes de galactomannane cationiques sont décrites plus particulièrement dans les brevets US 3 589 578 et 4 031 307 en particulier les gommes de guar contenant des groupements cationiques trialkylammonium. On utilise par exemple des gommes de guar modifiées par un sel (par ex. chlorure) de 2,3-époxypropyl triméthylammonium.
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(4) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "CARTARETINE F, F4 ou F8" par la société SANDOZ.
(6) les polymères obtenus par réaction d'une polyalkylène polyamine comportant deux groupements amine primaire et au moins un groupement amine secondaire avec un acide dicarboxylique notamment choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "HERCOSETT 57" par la société Hercules Inc. par la société HERCULES dans le cas du copolymère d'acide adipique/époxypropyl/diéthylènetriamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (I') :

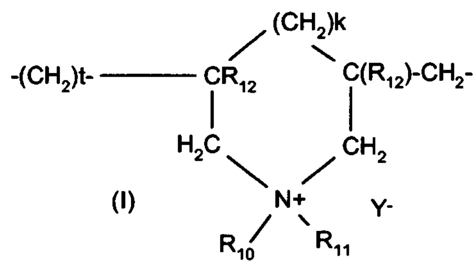

(I)

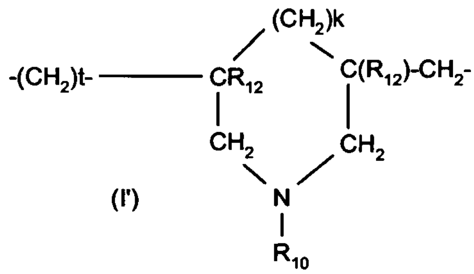

(I')

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. $R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "MERQUAT 100" par la société NALCO (et ses homologues de faibles masses molaires moyenne en poids) et les copolymères de chlorure de diallyldiméthylammonium et d'acrylamide.

(8) les polymères de diammonium quaternaire contenant des motifs récurrents répondant à la formule :

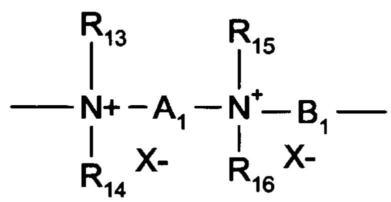

(II)

formule (II) dans laquelle :

> $R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
> A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
> A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
> dans lequel D désigne :
>
> > a) un reste de glycol de formule : -O-Z-O-, où Z désigne un radical hydrocarboné linéaire ou ramifié ou un groupement répondant à l'une des formules suivantes :
> >
> > - (CH2-CH2-O)x-CH2-CH2-
> > - [CH2-CH(CH3)-O]y-CH2-CH(CH3)-
> >
> > où x et y désignent un nombre entier de 1 à 4, représentant un degré de polymérisation défini et unique

ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;

De préférence, $X^-$ est un anion tel que le chlorure ou le bromure.

Ces polymères ont une masse molaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule :

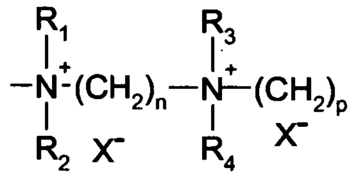
(a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

Un composé de formule (a) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(9) les polymères de polyammonium quaternaires comprenant des motifs de formule (III):

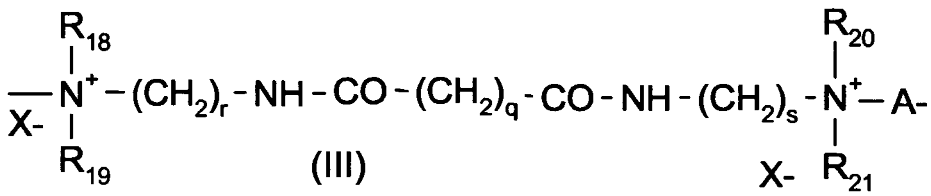
(III)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,
q est égal à 0 ou à un nombre entier compris entre 1 et 34,
X- désigne un anion tel qu'un halogènure,
A désigne un radical d'un dihalogénure ou représente de préférence $-CH_2-CH_2-O-CH_2-CH_2-$.

De tels composés sont notamment décrits dans la demande de brevet EP-A-122 324.

On peut par exemple citer parmi ceux-ci, les produits "Mirapol® A 15", "Mirapol® AD1 ", "Mirapol® AZ1" et "Mirapol® 175" vendus par la société Miranol.

(10) Les polymères quaternaires de vinylpyrrolidone et de vinylimidazole tels que par exemple les produits commercialisés sous les dénominations Luviquat® FC 905, FC 550 et FC 370 par la société B.A.S.F.

(11) Les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine, notamment les chitosanes ou leurs sels ;
les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90 % en poids, le pyrrolidone-carboxylate de chitosane commercialisé sous la dénomination KYTAMER® PC par la société AMERCHOL.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire,
dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société COGNIS.
Le composé vinylique peut être également un sel de dialkyldiallylammonium tel que le sel (par exemple chlorure) de diméthyldiallylammonium. Les copolymères d'acide acrylique et de ce dernier monomère sont proposés sous les appellations MERQUAT 280, MERQUAT 295 par la société NALCO.

(2) Les polymères comportant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer.

(3) Les polyaminoamides réticulés et alcoylés partiellement ou totalement dérivant de polyaminoamides de formule générale :

$$\left[ CO—R_4—CO—Z \right] \qquad (IV)$$

dans laquelle $R_4$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

$$—NH—\left[ (CH_2)_x—NH \right]_p— \qquad (V)$$

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (V) ci-dessus,

dans lequel x=2 et p=1 et qui dérive de l'éthylènediamine, ou le radical dérivant de la pipérazine :

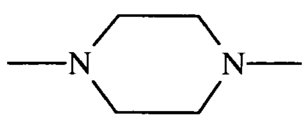

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.

(4) Les polymères comportant des motifs zwittérioniques de formule :

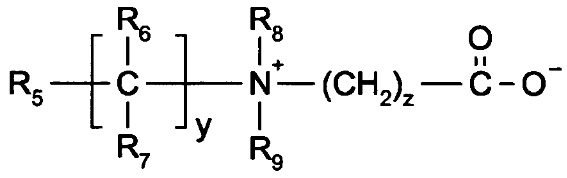

(VI)

dans laquelle $R_5$ désigne un groupement insaturé polymérisable tel qu'un groupement acrylate, méthacrylate, acrylamide ou méthacrylamide, y et z représentent un nombre entier de 1 à 3, $R_6$ et $R_7$ représentent un atome d'hydrogène, méthyle, éthyle ou propyle, $R_8$ et $R_9$ représentent un atome d'hydrogène ou un radical alkyle de telle façon que la somme des atomes de carbone dans $R_8$ et $R_9$ ne dépasse pas 10.

Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.

A titre d'exemple, on peut citer le copolymère de méthacrylate de butyle/méthacrylate de diméthyl-carboxyméthylammonio-éthyl.

(5) les polymères dérivés du chitosane comportant des motifs monomères répondant aux formules (VII), (VIII) et (IX) suivantes :

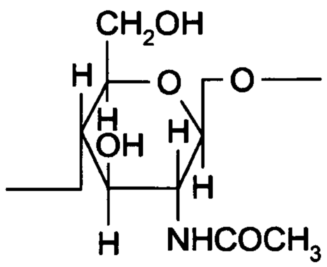

(VII)

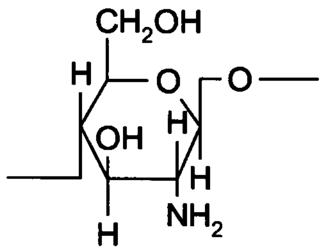

(VIII)

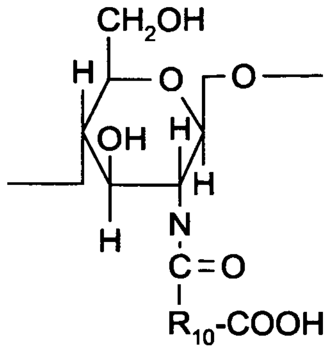

(IX)

le motif (VII) étant présent dans des proportions comprises entre 0 et 30%, le motif (VIII) dans des proportions comprises entre 5 et 50% et le motif (IX) dans des proportions comprises entre 30 et 90%, étant entendu que dans ce motif (IX), $R_{10}$ représente un radical de formule :

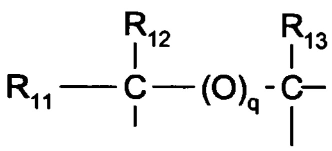

dans laquelle

si q=0, $R_{11}$, $R_{12}$ et $R_{13}$, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{11}$, $R_{12}$ et $R_{13}$ étant dans ce cas un atome d'hydrogène ;

ou si q=1, $R_{11}$, $R_{12}$ et $R_{13}$ représentent chacun un atome d'hydrogène, ainsi que les sels formés par ces composés avec des bases ou des acides.

(6) Les polymères dérivés de la N-carboxyalkylation du chitosane comme le N-carboxyméthyl chitosane ou le N-carboxybutyl chitosane.

(7) Les polymères répondant à la formule générale (X) tels que ceux décrits par exemple dans le brevet français 1 400 366 :

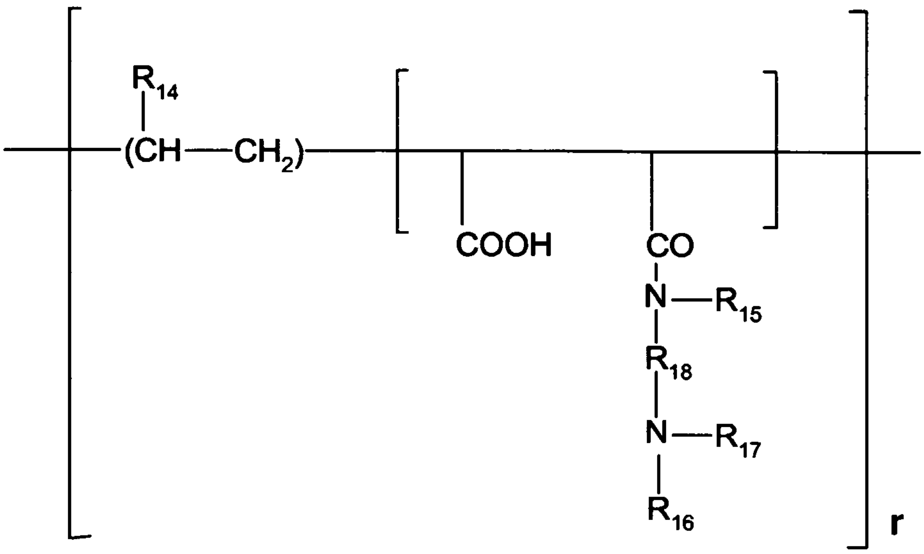

(X)

dans laquelle $R_{14}$ représente un atome d'hydrogène, un radical $CH_3O$, $CH_3CH_2O$, phényle, $R_{15}$ désigne l'hydrogène ou un radical alkyle inférieur tel que méthyle, éthyle, $R_{16}$ désigne l'hydrogène ou un radical alkyle inférieur tel que méthyle, éthyle, $R_{17}$ désigne un radical alkyle inférieur tel que méthyle, éthyle ou un radical répondant à la formule : $-R_{18}-N(R_{16})_2$, $R_{18}$ représentant un groupement $-CH_2-CH_2-$ , $-CH_2-CH_2-CH_2-$ , $-CH_2-CH(CH_3)-$ , $R_{16}$ ayant les significations mentionnées ci-dessus,
ainsi que les homologues supérieurs de ces radicaux et contenant jusqu'à 6 atomes de carbone.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- (XI)

où D désigne un radical

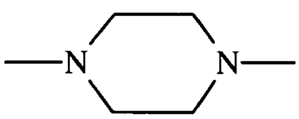

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- (XII)

où D désigne un radical

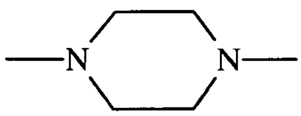

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères cationiques ou amphotères peuvent représenter de 0,001 % à 20 % en poids, de préférence de 0,01 % à 10% en poids et plus particulièrement de 0,02 à 5% en poids par rapport au poids total de la composition finale.

Selon un mode préféré de l'invention, les compositions peuvent comprendre en outre au moins une silicone différente des silicones aminées selon l'invention.

A titre de silicones utilisables dans les compositions de la présente invention, on peut notamment citer les silicones volatiles ou non, cycliques ou acycliques, ramifiées ou non, organomodifiées ou non, telles que décrites ci-dessous.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Selon l'invention, toutes les silicones peuvent être utilisées telle quelle ou sous forme de solutions, de dispersions, d'émulsions, de nanoémulsions ou de microémulsions.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et, de préférence, 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.

On peut également citer les cyclocopolymères du type diméthylsiloxane/méthylalkylsiloxane, tel que la "SILICONE VOLATILE FZ 3109" commercialisée par la société UNION CARBIDE, de structure chimique :

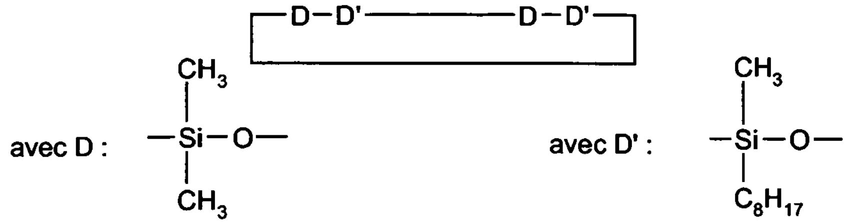

avec D :

avec D' :

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant de préférence une viscosité inférieure ou égale à $5.10^{-6} m^2/s$ à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.
Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est, par exemple, mesurée à 25°C selon la norme ASTM 445 Appendice C.
Parmi ces polyalkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple, l'huile 70 047 V 500 000 ;
- les huiles de la série MIRASIL® commercialisées par la société RHODIA
- les huiles de la série 200 de la société DOW CORNING telles que plus particulièrement la DC200 de viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA .
Dans cette classe de polyalkylsiloxanes, on peut également citer les produits commercialisés sous les dénominations "ABIL WAX® 9800 et 9801" par la société GOLDSCHMIDT qui sont des polyalkyl($C_1$-$C_{20}$)-siloxanes.
Les polyalkylarylsiloxanes sont particulièrement choisis parmi les polydiméthyl/méthylphénylsiloxanes, les polydiméthyldiphényl-siloxanes linéaires et/ou ramifiés de viscosité de $1.10^{-5}$ à $5.10^{-2}$ $m^2/s$ à 25°C.
Parmi ces polyalkylarylsiloxanes on peut citer, à titre d'exemple, les produits commercialisés sous les dénominations suivantes :

- les huiles SILBIONE® de la série 70 641 de RHODIA ;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.
On peut plus particulièrement citer les produits suivants :

- polydiméthylsiloxane
- les gommes polydiméthylsiloxane/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}$ $m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
$R_2SiO_{2/2}$, $R_3SiO_{1/2}$, $RSiO_{3/2}$ et $SiO_{4/2}$ dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en $C_1$-$C_4$, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :

- des groupements aminés,
- des groupements polyéthylèneoxy et/ou polypropylèneoxy comportant éventuellement des groupements alkyle en $C_6$-$C_{24}$ tels que les produits dénommés diméthicone-copolyol commercialisé par la société DOW CORNING sous la dénomination DC 1248 ou les huiles SILWET® L 722, L 7500, L 77, L 711 de la société UNION CARBIDE et l'alkyl($C_{12}$)-méthicone-copolyol commercialisée par la société DOW CORNING sous la dénomination Q2 5200 ;
- des groupements thiols, comme les produits commercialisés sous les dénominations "GP 72 A" et "GP 71" de GENESEE ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements hydroxylés, comme les polyorganosiloxanes à fonction hydroxyalkyle décrits dans la demande de brevet français FR-A-85 16334 .
- des groupements acyloxyalkyle tels que, par exemple, les polyorganosiloxanes décrits dans le brevet US-A-4957732.
- des groupements anioniques du type carboxylique comme, par exemple, dans les produits décrits dans le brevet EP 186 507 de la société CHISSO CORPORATION, ou de type alkyl-carboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL® S201" et "ABIL® S255".
- des groupements hydroxyacylamino, comme les polyorganosiloxanes décrits dans la demande EP 342 834. On peut citer, par exemple, le produit Q2-8413 de la société DOW CORNING.

Les silicones telles que décrites ci-dessus peuvent être utilisées seules ou en mélange, en une quantité comprise entre 0,01 et 20 % en poids, de préférence entre 0,05 et 5 % en poids par rapport au poids total de la composition.

Par milieu aqueux cosmétiquement acceptable, on entend un milieu compatible avec les matières kératiniques telles que notamment la peau, les cils, et les cheveux.

Le milieu aqueux cosmétiquement acceptable peut être constitué uniquement par de l'eau ou par un mélange d'eau et d'un solvant cosmétiquement acceptable tel qu'un alcool inférieur en $C_1$-$C_4$, tel que l'éthanol, l'isopropanol, le tertio-butanol, le n-butanol ; les alkylèneglycols comme le propylèneglycol, les éthers de polyols et leurs mélanges.

De préférence, la composition comprend de 50 à 95 % en poids d'eau par rapport au poids total de la composition et plus particulièrement de 70 à 90% en poids d'eau.

Les compositions de lavage selon l'invention présentent un pH final généralement compris entre 3 et 10. De préférence, ce pH est compris entre 4,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères d'acide acrylique tels que les copolymères d'acide acrylique et d'acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C16, les dérivés acylés à chaîne grasse tels que les monostéarates ou distéarates d'éthylène glycol ou de polyéthylèneglycol, les éthers à chaînes grasses (C10-C30) tels que par exemple le distéaryléther ou le 1-(hexadécyloxy)-2-octadécanol.

Les compositions conformes à l'invention peuvent également contenir en plus un ou plusieurs adjuvants choisis parmi parmi les synergistes de mousses tels que des 1,2-alcanediols en $C_{10}$-$C_{18}$ ou des alcanolamides gras dérivés de mono ou de diéthanolamine, les agents tensioactifs cationiques, les polymères anioniques ou non ioniques ou amphotères, les protéines, les hydrolysats de protéines, les céramides, les pseudocéramides, les acides gras à chaînes linéaires ou ramifiées notamment en $C_{12}$-$C_{40}$ tels que l'acide méthyl-18 eicosanoique, les huiles végétales, les huiles minérales ou synthétiques, les acides gras, les hydroxyacides, les vitamines, les provitamines telle que le panthénol, les filtres UV, les agents hydratants, les agents antipelliculaires ou antiséborrhéiques, les agents antichute des cheveux, les agents anti-radicaux libres, les agents opacifiants et leurs mélanges et tout autre additif classiquement utilisé dans le domaine cosmétique qui n'affecte pas les propriétés des compositions selon l'invention.

Les compositions de lavage selon l'invention peuvent bien entendu contenir en outre tous les adjuvants usuels rencontrés dans le domaine des shampooings, comme par exemple des parfums, des agents conservateurs, des sequestrants, des adoucissants, des colorants, des agents hydratants, des agents anti-pélliculaires ou anti-séborrhéiques, et autres.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions peuvent se présenter sous la forme de liquides, éventuellement épaissis, de crèmes ou de gel et elles conviennent principalement au lavage, au soin et/ou le coiffage des cheveux.

L'invention a pour objet l'utilisation en cosmétique d'une composition telle définie à l'une quelconque des revendications précédentes pour le nettoyage et/ou le soin et/ou le conditionnement et/ou le coiffage des cheveux.

L'invention a également pour objet l'utilisation d'une composition telle définie ci-dessus pour le nettoyage et/ou le démaquillage des matières kératiniques.

La présente invention concerne également un procédé de traitement cosmétique notamment de lavage et de conditionnement des matières kératiniques qui consiste à appliquer une quantité efficace d'une composition telle que décrite ci-dessus, sur lesdites matières kératiniques éventuellement mouillées , puis à effectuer un rinçage de préférence à l'eau après un éventuel temps de pose.

Selon un mode de réalisation préféré de l'invention, la composition peut être utilisée comme shampoing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Des exemples concrets, mais nullement limitatifs, illustrant l'invention vont maintenant être donnés.

**EXEMPLE 1**

On a réalisé une composition de shampooing conforme à l'invention :

| Composition | Invention | Comparatif |
|---|---|---|
| - Lauryléthersulfate de sodium (C12/C14 à 70/30) à 2.2 moles d'oxyde d'éthylène | 11,2 g M.A. | 11,2 g M.A. |
| - Cocoyl bétaïne | 5,1 g M.A. | 5,1 g M.A. |
| - Acide lauryl éther carboxylique (4.5 OE) | 3 g M.A. | 3 g M.A. |
| - Hydroxyéthylcellulose réticulée à l'épichlorhydrine et quaternisée par la triméthylamine, vendue sous la dénomination JR400 par la société AMERCHOL | 0,7 g | 0,7 g |
| - Propoxytetramethyl piperidinyl dimethicone C11-15 proposé sous la dénomination Jeesilc UAF par la société JEEN INTERNATIONAL | 1,5 g | - |
| - Polydiméthylsiloxane à groupements aminoéthyl iminobutyl commercialisé sous la dénomination DC2-8566 par la société DOW CORNING | - | 1,5 g |
| - Monoisopropanolamide d'acides de coprah | 2 g | 2 g |
| - Conservateurs, parfum | q.s. | q.s. |
| - Hydroxyde de sodium q.s. | pH 5,3 | pH 5,3 |
| - Eau déminéralisée q.s.p. | 100 g | 100 g |

On effectue un shampooing en appliquant la composition A sur des chevelures de cheveux sensibilisés préalablement mouillés. On fait mousser le shampooing, on laisse pauser pendant 10 minutes puis on rince abondamment à l'eau.

On procède selon le même mode opératoire que ci-dessus avec la composition comparative B.

Un panel d'experts a évalué l'aspect des cheveux mouillés et séchés.

Résultats :

- sur cheveux mouillés, les racines plus décollées pour les cheveux traités avec la composition A selon l'invention.
- sur cheveux séchés, le lissage visuel et au toucher est amélioré pour les cheveux traités avec la composition A selon l'invention.

Les cheveux traités avec le shampooing selon l'invention présentent des propriétés améliorées de lissage et de volume.

De plus, la coloration de la composition conforme à l'invention ne présente qu'une très faible évolution dans le temps, contrairement à la composition comparative dont le jaunissement est important.

**EXEMPLES 2 et 3**

On a réalisé des compositions de shampooing conformes à l'invention :

| Composition | Exemple 2 | Exemple 3 |
|---|---|---|
| - Lauryléthersulfate de sodium (C12/C14 à 70/30) à 2.2 moles d'oxyde d'éthylène | 11,2 g M.A. | 11,2 g M.A. |
| - Cocoyl bétaïne | 5,1 g M.A. | 5,1 g M.A. |
| - Acide lauryl éther carboxylique (4.5 OE) | 0,5 g M.A. | 0,5 g M.A. |
| - Hydroxyéthylcellulose réticulée à l'épichlorhydrine et quaternisée par la triméthylamine (JR400 de AMERCHOL) | 0,6 g | 0,6 g |
| - $\alpha,\omega$ diméthylpoly diméthyl, méthyl (3-(2,2,6,6- tetraméthylpipéridin-4-yloxy)propyl) siloxane, (Rhodorsil huile 21645 de RHODIA) | 1 g | - |
| - $\alpha,\omega$ diméthylpoly diméthyl, méthyl (3-(2,2,6,6- tetraméthylpipéridin-4-yloxy)propyl) siloxane, (Rhodorsil huile 21650 de RHODIA) | - | 1 g |

(suite)

| Composition | Exemple 2 | Exemple 3 |
|---|---|---|
| - Monoisopropanolamide d'acides de coprah | 0,5 g | 0,5 g |
| - Alcool cétylique oxyéthyléné (20 OE) oxypropyléné (5 OP) | 0,5 g | 0,5 g |
| - Conservateurs, parfum | q.s. | q.s. |
| - Hydroxyde de sodium q.s. | pH 5,3 | pH 5,3 |
| - Eau déminéralisée q.s.p. | 100 g | 100 g |

Ces compositions apportent du lissage visuel et au toucher aux cheveux sensibilisés, qui ont également du volume et sont brillants.

## Revendications

**1.** Composition de lavage des matières kératiniques **caractérisée en ce qu'**elle comprend, dans un milieu aqueux cosmétiquement acceptable, au moins une silicone aminée comprenant une fonction amine portée par un groupe stériquement encombré, à l'une au moins de ses extrémités et/ou sur des chaînes pendantes, ce groupe étant intégré dans un hétérocycle composé de 5 à 8 chaînons, les carbone en $\alpha$, et $\alpha'$ de l'atome d'azote étant totalement substitués par des atomes ou groupements différents de l'hydrogène, au moins un tensioactif détergent anionique, non ionique ou amphotère et au moins un polymère cationique, étant choisie parmi les polyorganosiloxanes ayant par mole au moins un motif de formule générale (I) :

$$\text{(I)} \qquad (R)_a(X)_bZSi(O)_{\frac{3-(a+b)}{2}}$$

dans laquelle :

- les symboles R sont identiques ou différents et représentent un radicalhydrocarboné monovalent choisi parmi les radicaux alkyles, linéaires ou ramifiés, ayant de 1 à 4 atomes de carbone, phényle, benzyle et trifluro-3,3,3 propyle;
- les symboles X sont identiques ou différents et représentent un radical monovalent choisi parmi un groupement hydroxyle et un radical alkoxy, linéaire ou ramifié, ayant de 1 à 3 atomes de carbone ;
- Z représente un radical monovalent comprenant une fonction amine portée par un groupe stériquement encombré, à savoir un ou plusieurs groupe(s) pipéridinyle(s) stériquement encombrés choisi parmi :

3) les radicaux de formule (II) :

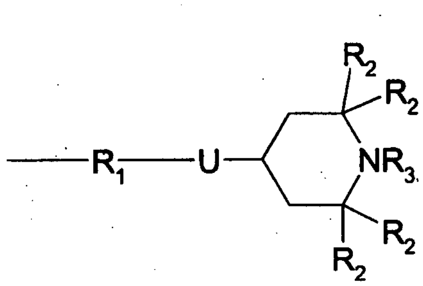

(II)

dans laquelle :

A) $R_1$ est un radical hydrocarboné divalent comprenant éventuellement un ou plusieurs hétéroatomes tels que O et N choisi parmi:

h) Les radicaux alkylènes, linéaires ou ramifiés, ayant de 2 à 18 atomes de carbone ;

i) Les radicaux alkylène-carbonyle dont la partie alkylène, linéaire ou ramifiée, comporte 2 à 20 atomes de carbone ;

j) Les radicaux alkylène-cyclohexylène dont la partie alkylène, linéaire ou ramifiée, comporte de 1 à 12 atomes de carbone et la partie cyclohexylène comporte un groupement OH et éventuellement 1 ou 2 radicaux alkyles ayant de 1 à 4 atomes de carbone ;

k)Les radicaux de formule $-R_4-O-R_5-$ dans laquelle les radicaux R4 et R5 identiques ou différents représentent des radicaux alkylènes ayant 1 à 12 atomes de carbone;

l) Les radicaux de formule $-R_4-O-R_5-$ dans laquelle les radicaux $R_4$ et $R_5$ ont les significations indiquées précédemment l'un d'entre eux ou les deux sont substitués par un ou deux groupement (s) OH ;

m) Les radicaux de formules $-R_4-COO-R_5-$ et $-R_4-OCO-R_5-$ dans lesquelles $R_4$ et $R_5$ ont les significations précédentes ;

n)Les radicaux de formule $-R_6-O-R_7-O-CO-R_8-$ dans laquelle $R_6$, $R_7$ et $R_8$, identiques ou différents, représentent des radicaux alkylènes ayant de 2 à 12 atomes de carbone et le radical $R_7$ est éventuellement substitué par un groupement hydroxyle ;

B) U représente -O- ou $-NR_9-$, $R_9$ étant un radical monovalent choisi parmi un atome d'hydrogène un radical alkyle, linéaire ou ramifié, ayant de 1 à 6 atomes de carbone; un radical divalent $-R_1-$ ayant la signification indiquée précédemment, l'un des liens valentiels étant relié à l'atome d'azote de $-NR_9-$, l'autre étant relié à un atome de silicium ;

C) $R_2$ sont des radicaux, identiques ou différents, choisis parmi les radicaux alkyles, linéaires ou ramifiés, ayant de 1 à 3 atomes de carbone et phényle ;

D) $R_3$ représente un atome d'hydrogène ou le radical $R_2$ ;

- a est un nombre choisi parmi 0, 1 et 2;
- b est un nombre choisi parmi 0, 1 et 2;
- la somme a+b est au plus égale à 2.

2. Composition selon la revendication 1, **caractérisée par le fait que** le ou les tensioactifs détergents sont choisis parmi les tensioactifs anioniques.

3. Composition selon la revendication 1 ou 2, **caractérisée par le fait que** ledit tensioactif est présent à une teneur pondérale allant de 4 % à 50 % en poids par rapport au poids total de la composition.

4. Composition selon la revendication 3, **caractérisée par le fait que** ladite teneur allant de 6 % à 35 % en poids par rapport au poids total de la composition.

5. Composition selon l'une quelconque des revendications 1 à 4, **caractérisée par le fait que** la silicone aminée comprend en outre au moins un autre motif siloxyle de formule (III) :

$$\text{(III)} \qquad (R)_c(X)_dVSi(O)_{\frac{3-(c+d)}{2}}$$

dans laquelle :

- les symboles R et X ont les mêmes significations que celles données ci avant à propos de la formule (I) ;
- le symbole V représente: un radical alkyle, linéaire ou ramifié, ayant de 5 à 20 atomes de carbone; un radical de formule $-(CH_2)_p-COOR_{12}$ dans laquelle p représente un nombre de 5 à 20 et $R_{12}$ représente un radical alkyle linéaire ou ramifié de 1 à 12 atomes de carbone ; un radical de formule $-(CH_2)_q-O-R_{13}$ dans laquelle q représente un nombre de 3 à 10 et $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un radical acyle ayant de 2 à 12 atomes de carbone ;
- c est un nombre choisi parmi 0, 1 et 2 ;
- d est un nombre choisi parmi 0, 1 et 2 ;
- la somme c + d est au plus égale à 2.

**6.** Composition selon l'une quelconque des revendications 1 à 5, **caractérisée par le fait que** la silicone aminée comprend en outre d'autre(s) motif(s) siloxyle(s) de formule (IV):

$$\text{(IV)} \quad (R)_e(X)_f Si(O)_{\frac{4-(e+f)}{2}}$$

dans laquelle :

- R et X ont les mêmes significations que celles données à propos de la formule (I) ;
- e est un nombre choisi parmi 0, 1, 2 et 3 ;
- f est un nombre choisi parmi 0, 1, 2 et 3 ;
- La somme e + f est au plus égale à 3.

**7.** Composition selon l'une quelconque des revendications 1 à 6, **caractérisée par le fait que** la silicone aminée est un polyorganosiloxane linéaire de formule moyenne (V) :

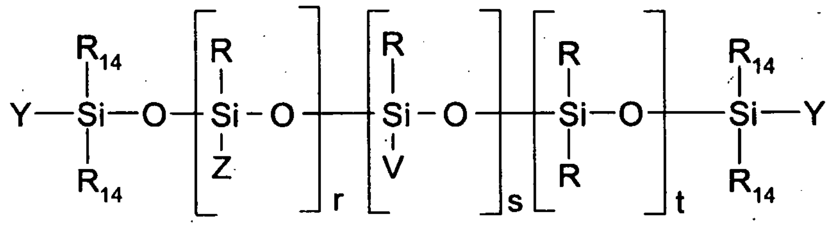

(V)

Dans laquelle:

- Les symboles R, Z et V ont les significations données ci avant à • propos des formules (I) et (III) ;
- Le symbole Y représente un radical monovalent choisi parmi les radicaux R, Z, V et X;
- Les symboles $R_{14}$ sont identiques ou différents et représentent un radical monovalent choisi parmi un radical R et un radical X tel que défini ci avant à propos de la formule (I) ;
- r, s et t sont égaux à zéro ou représentent des nombres entiers ou fractionnaires supérieurs à zéro, avec la condition supplémentaire selon laquelle si r = 0, au moins un des deux radicaux Y représente le radical Z.

**8.** Composition selon l'une quelconque des revendications 1 à 7, **caractérisée par le fait que** la silicone aminée est un polyorganosiloxane à motifs(s) de formules (I) + éventuellement (III) + éventuellement (IV) ou un polyorganosiloxane de formule (V) :

Ces polyorganosiloxanes ont en moyenne par mole : de 2 à 1600 atomes de silicium, de 1 à 100 radicaux Z tels que ceux ci-après définis et de 0 à 50 radicaux V tels que ceux ci-après définis ;

- et dans la structure duquel :

  - R est choisi parmi les radicaux méthyle, éthyle, n-propyle et isopropyle, de préférence méthyle ;
  - X est choisi parmi les radicaux hydroxyle, méthoxy et éthoxy ;
  - Z est choisi parmi les radicaux à groupes(s) pipéridinyle(s) :

    2) de formule (II) dans laquelle :

    A) $R_1$ est un radical hydrocarboné divalent comprenant éventuellement un ou plusieurs hétéroatomes tels que O et N choisi parmi :

    i) Les radicaux alkylènes, linéaires ou ramifiés, ayant de 2 à 18 atomes de carbone ;

j) Les radicaux alkylènes, linéaires ou ramifiés, ayant de 3 à 12 atomes de carbone;
k) Le radical $-(CH_2)_{10}-CO-$;
l) Les radicaux alkynèle-cyclohexylène dont la partie alkylène, linéaire ou ramifiée, comporte de 2 à 6 atomes de carbone et la partie cyclohylène comporte un groupement -OH et éventuellement 1 ou 2 subsistants alkyles ayant 1 à 4 atomes de carbone ;
m) Les radicaux de formule $-R_4-O-R_5-$ dans laquelle les radicaux $R_4$ et $R_5$ identiques ou différents représentent des radicaux alkylènes ayant 2 à 6 atomes de carbone ;
n) Les radicaux de formule $-R_4-O-R_5-$ dans laquelle les radicaux $R_4$ et $R_5$ ont les significations indiquées précédemment et $R_5$ est substitué par un groupe OH ;
o) Les radicaux de formules $-R_4-COO-R_5-$ et $-R_4-OCO-_{R5}-$ dans lesquelles $R_4$ et $R_5$ ont les significations précédentes ;
p) Les radicaux de formule $-R_6-O-R_7-O-CO-R_8-$ dans laquelle $R_6$, $R_7$, $R_8$, identiques ou différents, représentent des radicaux alkylènes ayant de 2 à 6 atomes de carbone et le radical $R_7$ est substitué par un groupement hydroxyde ;

B) U représente -O- ou $-NR_9-$ où $R_9$ est un radical alkyle, linéaire ou ramifié, ayant de 1 à 4 atomes de carbone;
C) R2 représente un radical méthyle;

• V est choisi parmi: un radical alkyle, linéaire ou ramifié, ayant 5 à 18 atomes de carbone; un radical de formule $-(CH_2)_{10}-COO-R_{12}$ dans laquelle $R_{12}$ représente un radical alkyle , linéaire de 1 à 6 atomes de carbone, un radical de formule $-(CH_2)_3-O-R_{13}$, $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un groupement acyle ayant de 2 à 6 atomes de carbone.

**9.** Composition selon la revendication 8, **caractérisée par le fait que** la silicone aminée est un polydiorganosiloxane linéaire de formule (V) présentant les valeurs suivantes pour les symboles r, s et t :

• r est un nombre entier ou fractionnaire allant de 0 à 98 avec la condition selon laquelle si r = 0, au moins un des deux radicaux Y représente le radical Z
• s est un nombre entier ou fractionnaire allant de 0 à 48 ;
• t est un nombre entier ou fractionnaire allant de 0 à 1598 :
• la somme r + s + t est un nombre entier ou fractionnaire allant de 0 à 1598.

**10.** Composition selon l'une quelconque des revendications 1 à 9, **caractérisée par le fait que** la silicone aminée est un polyorganosiloxane ayant la structure suivante :

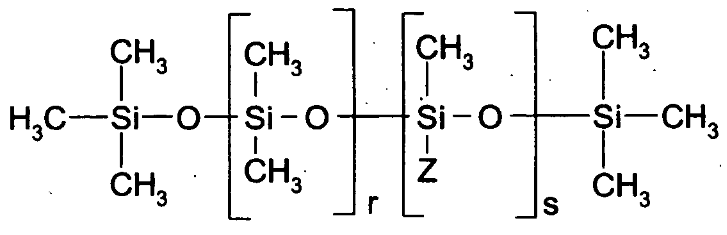

avec Z =

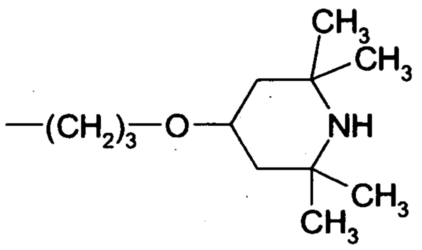

s varie de 1 à 50, de préférence de 2 à 30 ;
r varie de 50 à 2000, de préférence de 500 à 1700.

**11.** Composition selon l'une quelconque des revendications 1 à 10, **caractérisée par le fait que** la silicone aminée est présente dans des concentrations allant de 0,001 % à 10 % en poids, de préférence de 0,01 % à 5 % en poids, et encore plus préférentiellement de 0,1 % à 3 % en poids, du poids total de la composition finale.

**12.** Composition selon l'une quelconque des revendications 1 à 11, **caractérisée par le fait que** le polymère cationique est choisi parmi :

(1) les homopolymères ou copolymères dérivés d'esters ou d'amides acryliques ou méthacryliques et comportant au moins un des motifs de formules suivantes:

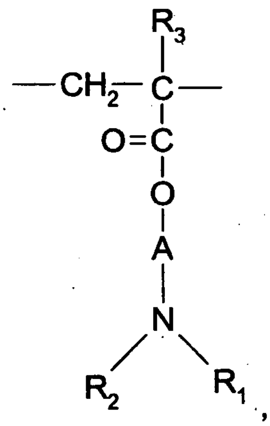

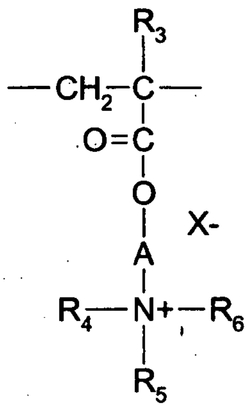

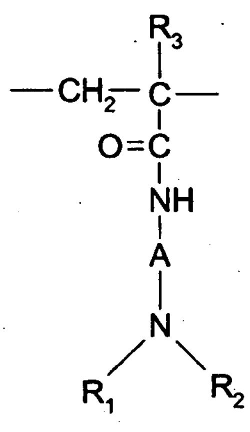

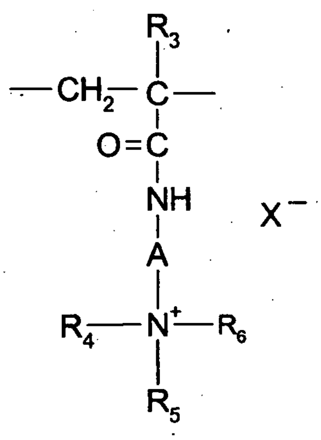

dans lesquelles:

$R_3$, identiques ou différents, désignent un atome d'hydrogène où un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ; .
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.

(2) Les polysaccharides cationiques
(3) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quatemisation de ces polymères
(4) les polyaminoamides solubles dans l'eau préparés en particulier par polycondensation d'un composé acide avec une polyamine ;
(5) les dérivés de polyaminoamides résultant de la condensation de polyalcoylènes polyamines avec des acides polycarboxyliques suivie d'une alcoylation par des agents bifonctionnels
(6) les polymères obtenus par réaction d'une polyalkylène polyamine comportant deux groupements amine primaire et au moins un groupement amine secondaire avec un acide dicarboxylique
(7) les cyclopolymères d'alkyl diallyl amine ou de dialkyl diallyl ammonium
(8) les polymères de diammonium quaternaire contenant des motifs récurrents répondant à la formule :

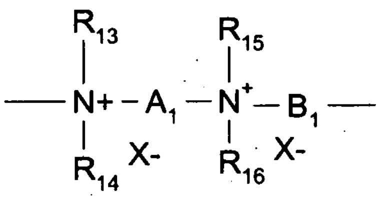
(II)

formule (II) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$ et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
$X^-$ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

$X^-$ est un anion

(9) les polymères de polyammonium quaternaires comprenant des motifs de formule (III):

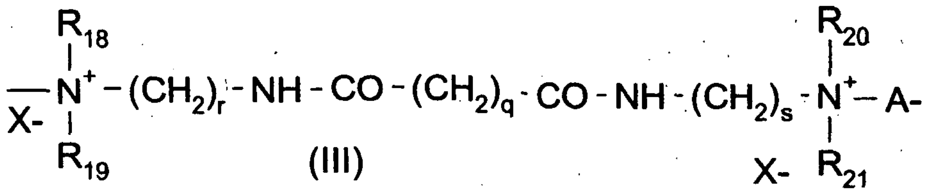

(III)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,
q est égal à 0 ou à un nombre entier compris entre 1 et 34,
$X^-$ désigne un anion tel qu'un halogènure,
A désigne un radical d'un dihalogénure ou représente de préférence $-CH_2-CH_2-O-CH_2-CH_2-$.

(10) Les polymères quaternaires de vinylpyrrolidone et de vinylimidazole,
(11) Les polymères réticulés de sels de méthacryloyloxyalkyl(C1-C4) trialkyl(C1-C4)ammonium,
(12) lesprotéines cationiques ou des hydrolysats de protéines cationiques, des polyalkylèneimines, des polymères contenant des motifs vinylpyridine ou vinylpyridinium, des condensats de polyamines et d'épichlorhydrine, des polyuréylènes quaternaires et les dérivés de la chitine;

**13.** Composition selon la revendication 12 **caractérisée par** le fait le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques non cellulosiques, les copolymères quaternaires de vinylpyrrolidone et de sel de vinyl imidazole.

**14.** Composition selon l'une quelconque des revendications 11 à 13, **caractérisée en ce que** le ou les polymères cationiques représentent de 0,001 % à 20 % en poids, de préférence de 0,01 % à 10% en poids, par rapport au poids total de la composition finale.

**15.** Composition selon l'une quelconque des revendications précédentes, **caractérisée par le fait que** la composition comprend en outre au moins une silicone différente des silicones aminées comprenant une fonction amine portée par un groupe stériquement encombré.

**16.** Composition selon la revendication précédente, **caractérisée par le fait que** la silicone différente des silicones aminées est utilisée en une quantité comprise entre 0,01 et 20 % en poids, de préférence entre 0,1 et 5 % en poids

par rapport au poids total de la composition.

**17.** Composition selon l'une quelconque des revendications précédentes, **caractérisée par le fait qu'**elle présente un pH compris entre 3 et 10.

**18.** Composition selon l'une quelconque des revendications 1 à 17, **caractérisée par le fait qu'**elle contient en plus un ou plusieurs adjuvants choisis parmi les agents tensioactifs cationiques, les polymères anioniques ou non ioniques ou amphotères, les protéines, les hydrolysats de protéines, les céramides, les pseudocéramides, les huiles végétales, les huiles minérales ou synthétiques, les acides gras, les hydroxyacides, les vitamines, les provitamines telle que le panthénol, les filtres UV, les agents hydratants, les agents antipelliculaires ou antiséborrhéiques, les agents antichute des cheveux, les agents anti-radicaux libres, les agents opacifiants et leurs mélanges.

**19.** Utilisation en cosmétique d'une composition telle définie à l'une quelconque des revendications précédentes pour le nettoyage et/ou le soin et/ou le conditionnement et/ou le coiffage des cheveux.

**20.** Utilisation d'une composition telle définie dans l'une quelconque des revendications 1 à 18 pour le nettoyage et/ou le démaquillage des matières kératiniques.

**21.** Procédé de lavage et de conditionnement des matières kératiniques telles que les cheveux consistant à appliquer sur lesdites matières éventuellement mouillées une quantité efficace d'une composition telle que définie dans l'une quelconque des revendications 1 à 18 puis à effectuer un rinçage de préférence à l'eau après un éventuel temps de pause.

## Claims

**1.** Composition for washing keratin materials, **characterized in that** it comprises, in a cosmetically acceptable aqueous medium, at least one amino silicone comprising an amine function borne by a sterically hindered group on at least one of its ends and/or on side chains, this group being integrated in a 5- to 8-membered heterocycle, the carbons $\alpha$ and $\alpha'$ to the nitrogen atom being totally substituted with atoms or groups other than hydrogen, at least one anionic, nonionic or amphoteric detergent surfactant and at least one cationic polymer, the amino silicone being chosen from polyorganosiloxanes containing, per mole, at least one unit of general formula (I):

$$\text{(I)} \qquad (R)_a(X)_bZSi(O)_{\frac{3-(a+b)}{2}}$$

in which:

- the symbols R are identical or different and represent a monovalent hydrocarbon-based radical chosen from linear or branched alkyl radicals containing from 1 to 4 carbon atoms, phenyl, benzyl and 3,3,3-trifluoropropyl;
- the symbols X are identical or different and represent a monovalent radical chosen from a hydroxyl
- group and a linear or branched alkoxy radical containing from 1 to 3 carbon atoms;
- Z represents a monovalent radical comprising an amine function borne by a sterically hindered group, namely one or more sterically hindered piperidyl group(s) chosen from:

3) the radicals of formula (II):

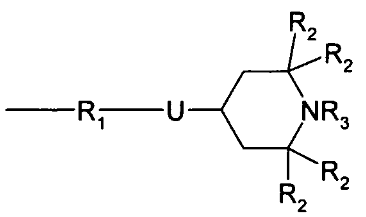

(II)

in which:

A) $R_1$ is a divalent hydrocarbon-based radical optionally comprising one or more heteroatoms such as O and N, chosen from:

h) linear or branched alkylene radicals containing from 2 to 18 carbon atoms;
i) alkylene-carbonyl radicals in which the linear or branched alkylene portion contains 2 to 20 carbon atoms;
j) alkylene-cyclohexylene radicals in which the linear or branched alkylene portion contains 1 to 12 carbon atoms and the cyclohexylene portion comprises an OH group and optionally one or two alkyl radicals containing from 1 to 4 carbon atoms;
k) the radicals of formula $-R_4-O-R_5-$ in which the radicals $R_4$ and $R_5$, which may be identical or different, represent alkylene radicals containing 1 to 12 carbon atoms;
l) the radicals of formula $-R_4-O-R_5-$ in which the radicals $R_4$ and $R_5$ have the same meanings as above and one or both of them is (are) substituted with one or two OH group(s);
m) the radicals of formulae $-R_4-COO-R_5-$ and $-R_4-OCO-R_5-$ in which the radicals $R_4$ and $R_5$ have the above meanings;
n) the radicals of formula $-R_6-O-R_7-O-CO-R_8-$ in which $R_6$, $R_7$ and $R_8$, which may be identical or different, represent alkylene radicals containing from 2 to 12 carbon atoms and the radical $R_7$ is optionally substituted with a hydroxyl group;

B) U represents -O- or $-NR_9-$, $R_9$ being a monovalent radical chosen from a hydrogen atom, a linear or branched alkyl radical containing from 1 to 6 carbon atoms; a divalent radical $-R_1-$ having the meaning given above, one of the valency bonds being linked to the nitrogen atom of $-NR_9-$, the other being linked to a silicon atom;
C) $R_2$ are identical or different radicals chosen from linear or branched alkyl radicals containing from 1 to 3 carbon atoms and phenyl;
D) $R_3$ represents a hydrogen atom or a radical $R_2$;

- a is a number chosen from 0, 1 and 2;
- b is a number chosen from 0, 1 and 2;
- the sum a + b is not more than 2.

**2.** Composition according to Claim 1, **characterized in that** the detergent surfactant(s) is (are) chosen from anionic surfactants.

**3.** Composition according to Claim 1 or 2, **characterized in that** the said surfactant is present in a weight ratio ranging from 4% to 50% by weight relative to the total weight of the composition.

**4.** Composition according to Claim 3, **characterized in that** the said content ranges from 6% to 35% by weight relative to the total weight of the composition.

**5.** Composition according to any one of Claims 1 to 4, **characterized in that** the amino silicone also comprises at least one other siloxyl unit of formula (III):

$$\text{(III)} \qquad (R)_c(X)_d VSi(O)_{\frac{3-(c+d)}{2}}$$

in which:

- the symbols R and X have the same meanings as those given above with respect to formula (I);
- the symbol V represents: a linear or branched alkyl radical containing from 5 to 20 carbon atoms; a radical of formula $-(CH_2)_p-COOR_{12}$ in which p represents a number from 5 to 20 and $R_{12}$ represents a linear or branched alkyl radical of 1 to 12 carbon atoms; a radical of formula $-(CH_2)_q-O-R_{13}$ in which q represents a number from 3 to 10 and $R_{13}$ represents a hydrogen atom, an ethylene oxide, a mono- or polyoxyethylene group, a mono- or polyoxypropylene group, a mixed chain consisting of ethylene oxide and propylene oxide units or an acyl

radical containing from 2 to 12 carbon atoms;
• c is a number chosen from 0, 1 and 2;
• d is a number chosen from 0, 1 and 2;
• the sum c + d is not more than 2.

**6.** Composition according to any one of Claims 1 to 5, **characterized in that** the amino silicone also comprises other siloxyl units of formula (IV):

$$\text{(IV)} \qquad (R)_e(X)_fSi(O)_{\frac{4-(e+f)}{2}}$$

in which

• R and X have the same meanings as those given with respect to formula (I);
• e is a number chosen from 0, 1, 2 and 3;
• f is a number chosen from 0, 1, 2 and 3;
• the sum e + f is not more than 3.

**7.** Composition according to any one of Claims 1 to 6, **characterized in that** the amino silicone is a linear polyorganosiloxane of mean formula (V):

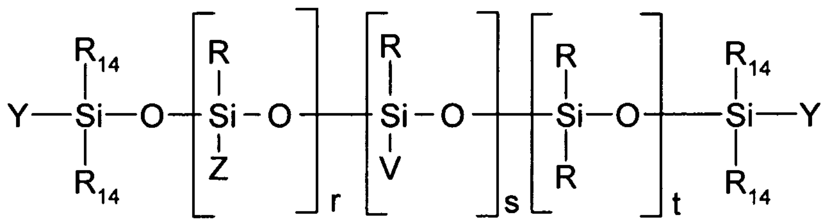

(V)

in which:

• the symbols R, Z and V have the meanings given above with respect to formulae (I) and (III);
• the symbol Y represents a monovalent radical chosen from the radicals R, Z, V and X;
• the symbols $R_{14}$ are identical or different and represent a monovalent radical chosen from a radical R and a radical X as defined above with respect to formula (I);
• r, s and t are equal to zero or represent integers or fractional numbers greater than zero, with the additional condition that if r = 0, at least one of the two radicals Y represents the radical Z.

**8.** Composition according to any one of Claims 1 to 7, **characterized in that** the amino silicone is a polyorganosiloxane containing units of formulae (I) + optionally (III) + optionally (IV) or a polyorganosiloxane of formula (V):

these polyorganosiloxanes containing on average, per mole: from 2 to 1600 silicon atoms, from 1 to 100 radicals Z such as those defined below and from 0 to 50 radicals V such as those defined below;
- and in the structure of which:

• R is chosen from methyl, ethyl, n-propyl and isopropyl radicals, preferably methyl;
• X is chosen from hydroxyl, methoxy and ethoxy radicals;
• Z is chosen from radicals containing piperidyl group(s):

2) of formula (II) in which:

A) $R_1$ is a divalent hydrocarbon-based radical optionally comprising one or more heteroatoms such as O and N, chosen from:

i) linear or branched alkylene radicals containing from 2 to 18 carbon atoms;
j) linear or branched alkylene radicals containing 3 to 12 carbon atoms;
k) the radical - $(CH_2)_{10}$-CO-;
l) alkylene-cyclohexylene radicals in which the linear or branched alkylene portion contains from 2 to 6 carbon atoms and the cyclohexylene portion contains an -OH group and optionally one or two alkyl substituents containing 1 to 4 carbon atoms;
m) the radicals of formula -$R_4$-O-$R_5$- in which the radicals $R_4$ and $R_5$, which may be identical or different, represent alkylene radicals containing from 2 to 6 carbon atoms;
n) the radicals of formula -$R_4$-O-$R_5$- in which the radicals $R_4$ and $R_5$ have the meanings given above and $R_5$ is substituted with an OH group;
o) the radicals of formulae -$R_4$-COO-$R_5$- and -$R_4$-OCO-$R_5$- in which the radicals $R_4$ and $R_5$ have the above meanings;
p) the radicals of formula -$R_6$-O-$R_7$-O-CO-$R_8$- in which $R_6$, $R_7$ and $R_8$, which may be identical or different, represent alkylene radicals containing from 2 to 6 carbon atoms and the radical $R_7$ is substituted with a hydroxyl group;

B) U represents -O- or -$NR_9$-, in which $R_9$ is a linear or branched alkyl radical containing from 1 to 4 carbon atoms;
C) $R_2$ represents a methyl radical;

• V is chosen from: a linear or branched alkyl radical containing from 5 to 18 carbon atoms; a radical of formula -$(CH_2)_{10}$-COO-$R_{12}$ in which $R_{12}$ represents a linear alkyl radical of 1 to 6 carbon atoms, a radical of formula -$(CH_2)_3$-O-$R_{13}$, in which $R_{13}$ represents a hydrogen atom, an ethylene oxide, a mono- or polyoxyethylene group, a mono- or polyoxypropylene group, a mixed chain consisting of ethylene oxide and propylene oxide units or an acyl group containing from 2 to 6 carbon atoms.

**9.** Composition according to Claim 8, **characterized in that** the amino silicone is a linear polydiorganosiloxane of formula (V) having the following values for the symbols r, s and t:

• r is an integer or fractional number ranging from 0 to 98, with the condition that if r = 0, at least one of the two radicals Y represents a radical Z;
• s is an integer or fractional number ranging from 0 to 48;
• t is an integer or fractional number ranging from 0 to 1598;
• the sum r + s + t is an integer or fractional number ranging from 0 to 1598.

**10.** Composition according to any one of Claims 1 to 9, **characterized in that** the amino silicone is a polyorganosiloxane having the following structure:

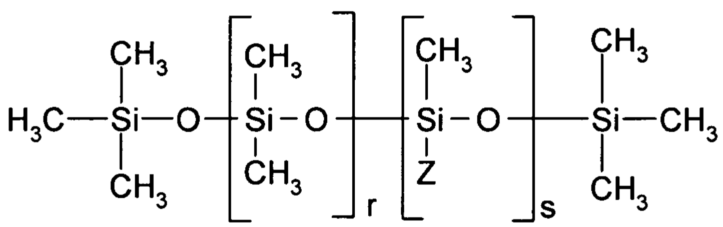

with Z =

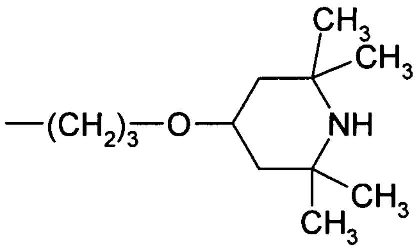

s ranges from 1 to 50 and preferably from 2 to 30,
r ranges from 50 to 2000 and preferably from 500 to 1700.

**11.** Composition according to any one of Claims 1 to 10, **characterized in that** the amino silicone is present in concentrations ranging from 0.001% to 10% by weight, preferably from 0.01% to 5% by weight and even more preferentially from 0.1% to 3% by weight relative to the total weight of the final composition.

**12.** Composition according to any one of Claims 1 to 11, **characterized in that** the cationic polymer is chosen from:

(1) homopolymers or copolymers derived from acrylic or methacrylic esters or amides and comprising at least one of the units of the following formulae:

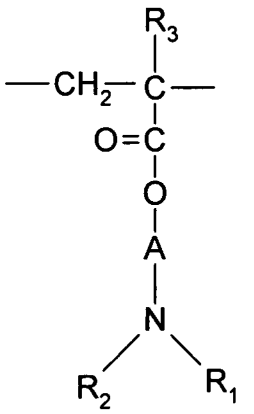 , 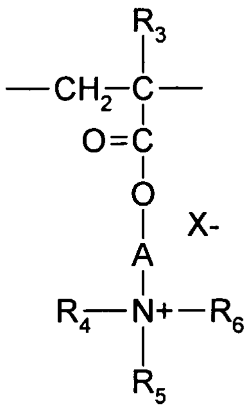

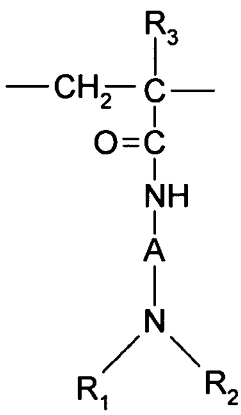 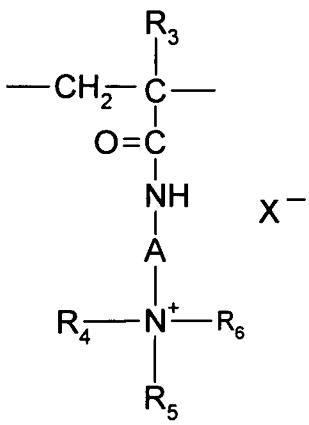

in which:

$R_3$, which may be identical or different, denote a hydrogen atom or a $CH_3$ radical;
A, which may be identical or different, represent a linear or branched alkyl group of 1 to 6 carbon atoms,

preferably 2 or 3 carbon atoms, or a hydroxyalkyl group of 1 to 4 carbon atoms;

$R_4$, $R_5$ and $R_6$, which may be identical or different, represent an alkyl group containing from 1 to 18 carbon atoms or a benzyl radical and preferably an alkyl group containing from 1 to 6 carbon atoms;

$R_1$ and $R_2$, which may be identical or different, represent hydrogen or an alkyl group containing from 1 to 6 carbon atoms, and preferably methyl or ethyl;

$X^-$ denotes an anion derived from a mineral or organic acid, such as a methosulfate anion or a halide such as chloride or bromide;

(2) cationic polysaccharides;

(3) polymers consisting of piperazinyl units and of divalent alkylene or hydroxyalkylene radicals containing straight or branched chains, optionally interrupted by oxygen, sulfur or nitrogen atoms or by aromatic or heterocyclic rings, and also the oxidation and/or quaternization products of these polymers;

(4) water-soluble polyamino amides prepared in particular by polycondensation of an acidic compound with a polyamine;

(5) polyamino amide derivatives resulting from the condensation of polyalkylene polyamines with polycarboxylic acids followed by alkylation with bifunctional agents;

(6) polymers obtained by reaction of a polyalkylene polyamine containing two primary amine groups and at least one secondary amine group with a dicarboxylic acid;

(7) cyclopolymers of alkyldiallylamine or of dialkyldiallylammonium;

(8) diquaternary ammonium polymers containing repeating units corresponding to the formula:

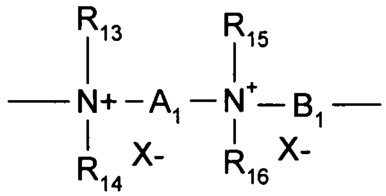

(II)

in which formula (II):

$R_{13}$, $R_{14}$, $R_{15}$ and $R_{16}$, which may be identical or different, represent aliphatic, alicyclic or arylaliphatic radicals containing from 1 to 20 carbon atoms or lower hydroxyalkylaliphatic radicals, or alternatively $R_{13}$, $R_{14}$, $R_{15}$ and $R_{16}$, together or separately, constitute, with the nitrogen atoms to which they are attached, heterocycles optionally containing a second heteroatom other than nitrogen, or alternatively $R_{13}$, $R_{14}$, $R_{15}$ and $R_{16}$ represent a linear or branched $C_1$-$C_6$ alkyl radical substituted with a nitrile, ester, acyl or amide group or a group $-CO-O-R_{17}-D$ or $-CO-NH-R_{17}-D$ where $R_{17}$ is an alkylene and D is a quaternary ammonium group;

$A_1$ and $B_1$ represent polymethylene groups containing from 2 to 20 carbon atoms, which groups may be linear or branched, saturated or unsaturated, and which may contain, linked to or intercalated in the main chain, one or more aromatic rings or one or more oxygen or sulfur atoms or sulfoxide, sulfone, disulfide, amino, alkylamino, hydroxyl, quaternary ammonium, ureido, amide or ester groups, and

$X^-$ denotes an anion derived from a mineral or organic acid;

$A_1$, $R_{13}$ and $R_{15}$ can form, with the two nitrogen atoms to which they are attached, a piperazine ring; in addition, if $A_1$ denotes a linear or branched, saturated or unsaturated alkylene or hydroxyalkylene radical, $B_1$ can also denote a group $(CH_2)_n-CO-D-OC-(CH_2)_n-$;

in which D denotes:

a) a glycol residue of formula: -O-Z-O-, where Z denotes a linear or branched hydrocarbon-based radical or a group corresponding to one of the following formulae:

$$-(CH_2-CH_2-O)_x-CH_2-CH_2-$$

$$-[CH_2-CH(CH_3)-O]_y-CH_2-CH(CH_3)-$$

where x and y denote an integer from 1 to 4, representing a defined and unique degree of polymerization

or any number from 1 to 4 representing an average degree of polymerization;
b) a bis-secondary diamine residue such as a piperazine derivative;
c) a bis-primary diamine residue of formula: -NH-Y-NH-, where Y denotes a linear or branched hydrocarbon-based radical, or alternatively the divalent radical

$$-CH_2-CH_2-S-S-CH_2-CH_2-;$$

d) a ureylene group of formula: -NH-CO-NH-;

$X^-$ is an anion;
(9) polyquaternary ammonium polymers consisting of units of formula (III):

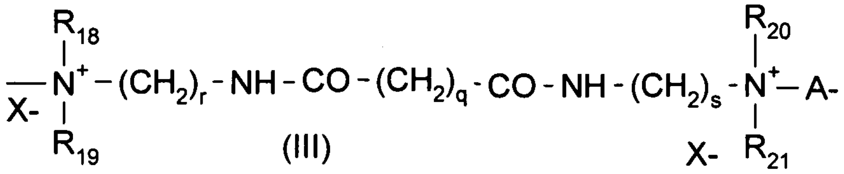

(III)

in which formula:

$R_{18}$, $R_{19}$, $R_{20}$ and $R_{21}$, which may be identical or different, represent a hydrogen atom or a methyl, ethyl, propyl, β-hydroxyethyl, β-hydroxypropyl or $-CH_2CH_2(OCH_2CH_2)_pOH$ radical,
where p is equal to 0 or to an integer between 1 and 6, with the proviso that $R_{18}$, $R_{19}$, $R_{20}$ and $R_{21}$ do not simultaneously represent a hydrogen atom,
r and s, which may be identical or different, are integers ranging from 1 to 6,
q is equal to 0 or to an integer ranging from 1 to 34,
$X^-$ denotes an anion such as a halide,
A denotes a dihalide radical or preferably represents $-CH_2-CH_2-o-CH_2-CH_2-$;

(10) quaternary polymers of vinylpyrrolidone and of vinylimidazole;
(11) crosslinked polymers of methacryloyloxy($C_1$-$C_4$)alkyltri($C_1$-$C_4$)alkylammonium salts;
(12) cationic proteins or cationic protein hydrolysates, polyalkyleneimines, polymers containing vinylpyridine or vinylpyridinium units, condensates of polyamines and of epichlorohydrin, polyquaternary ureylenes and chitin derivatives.

**13.** Composition according to Claim 12, **characterized in that** the cationic polymer is chosen from quaternary cellulose ether derivatives, diallyldimethylammonium salt homopolymers, and copolymers of a diallyldimethylammonium salt and of acrylamide, non-cellulose-based cationic polysaccharides, and quaternary copolymers of vinylpyrrolidone and of a vinylimidazole salt.

**14.** Composition according to any one of Claims 11 to 13, **characterized in that** the cationic polymer(s) represent (s) from 0.001% to 20% by weight and preferably from 0.01% to 10% by weight relative to the total weight of the final composition.

**15.** Composition according to any one of the preceding claims, **characterized in that** the composition also comprises at least one silicone other than amino silicones comprising an amine function borne by a sterically hindered group.

**16.** Composition according to the preceding claim, **characterized in that** the silicone other than amino silicones is used in an amount of between 0.01% and 20% by weight and preferably between 0.1% and 5% by weight relative to the total weight of the composition.

**17.** Composition according to any one of the preceding claims, **characterized in that** it has a pH of between 3 and 10.

**18.** Composition according to any one of Claims 1 to 17, **characterized in that** it also contains one or more adjuvants chosen from cationic surfactants, anionic, nonionic or amphoteric polymers, proteins, protein hydrolysates, ceramides, pseudoceramides, plant oils, mineral or synthetic oils, fatty acids, hydroxy acids, vitamins, provitamins such as panthenol, UV-screening agents, moisturizers, antidandruff or anti-seborrhoeic agents, hair-loss counteractants, free-radical scavengers and opacifiers, and mixtures thereof.

**19.** Cosmetic use of a composition as defined in any one of the preceding claims, for cleansing and/or caring for and/or conditioning and/or styling the hair.

**20.** Use of a composition as defined in any one of Claims 1 to 18, for cleansing and/or removing makeup from keratin materials.

**21.** Process for washing and conditioning keratin materials such as the hair, which consists in applying to the said optionally moistened materials an effective amount of a composition as defined in any one of Claims 1 to 18, and then in rinsing it out, preferably with water, after an optional leave-in time.

**Patentansprüche**

**1.** Zusammensetzung für die Reinigung von Keratinsubstanzen, **dadurch gekennzeichnet, dass** sie in einem kosmetisch akzeptablen, wässrigen Medium mindestens ein aminiertes Silicon, das an mindestens einem seiner Enden und/oder an Seitenketten eine von einer sterisch gehinderten Gruppe getragene Aminofunktion aufweist, wobei diese Gruppe in einen 5- bis 8-gliedrigen Heterocyclus integriert ist und die Kohlenstoffatome in α- und α'-Stellung des Stickstoffatoms vollständig mit Atomen oder Gruppen substituiert sind, die von Wasserstoff verschieden sind, mindestens einen anionischen, nichtionischen oder amphoteren reinigenden grenzflächenaktiven Stoff und mindestens ein kationisches Polymer enthält, wobei das aminierte Silicon unter den Polyorganosiloxanen ausgewählt ist, die pro Mol mindestens eine Einheit der folgenden allgemeinen Formel (I) aufweisen:

$$\text{(I)} \qquad (R)_a(X)_bZSi(O)_{\frac{3-(a+b)}{2}}$$

in der Formel:

- die Symbole R sind gleich oder verschieden und bedeuten eine einwertige Kohlenwasserstoffgruppe, die unter den linearen oder verzweigten Alkylgruppen mit 1 bis 4 Kohlenstoffatomen, Phenyl, Benzyl und 3,3,3-Trifluorpropyl ausgewählt ist;
- die Symbole X sind gleich oder verschieden und bedeuten eine einwertige Gruppe, die unter einer Hydroxygruppe und einer linearen oder verzweigten Alkoxygruppe mit 1 bis 3 Kohlenstoffatomen ausgewählt ist;
- Z ist eine einwertige Gruppe, die eine Aminofunktion aufweist, die von einer sterisch gehinderten Gruppe getragen wird, d.h. eine oder mehrere sterisch gehinderte(n) Piperidinylgruppe(n), die ausgewählt sind unter:

den Gruppen der folgenden Formel (II):

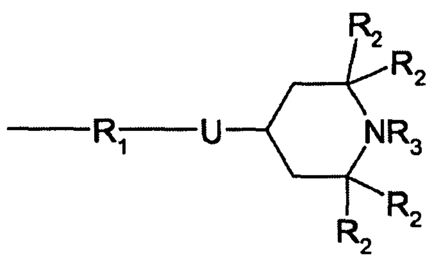

(II)

in der Formel:

A) $R_1$ ist eine zweiwertige Kohlenwasserstoffgruppe, die gegebenenfalls ein oder mehrere Heteroatome

wie O und N enthält und die ausgewählt ist unter:

h) linearen oder verzweigten Alkylengruppen mit 2 bis 18 Kohlenstoffatomen;
i) Alkylen-carbonylgruppen, deren linearer oder verzweigter Alkylenteil 2 bis 20 Kohlenstoffatome aufweist;
j) Alkylen-cyclohexylengruppen, deren linearer oder verzweigter Alkylenteil 1 bis 12 Kohlenstoffatome aufweist und deren Cyclohexylenteil eine OH-Gruppe und gegebenenfalls 1 oder 2 Alkylgruppen mit 1 bis 4 Kohlenstoffatomen umfasst;
k) den Gruppen der Formel $-R_4-O-R_5-$, worin die Gruppen $R_4$ und $R_5$, die gleich oder verschieden sind, Alkylengruppen mit 1 bis 12 Kohlenstoffatomen bedeuten;
l) den Gruppen der Formel $-R_4-O-R_5-$, worin die Gruppen $R_4$ und $R_5$ die oben angegebenen Bedeutungen aufweisen, wobei eine Gruppe oder beide Gruppen mit einer oder mit zwei Gruppe(n) OH substituiert sind;
m) den Gruppen der Formel $-R_4-COO-R_5-$ und $-R_4-OCO-R_5$, worin die Gruppen $R_4$ und $R_5$ die oben angegebenen Bedeutungen aufweisen;
n) den Gruppen der Formel $-R_6-O-R_7-O-CO-R_8-$, worin die Gruppen $R_6$, $R_7$ und $R_8$, die gleich oder verschieden sind, Alkylengruppen mit 2 bis 12 Kohlenstoffatomen bedeuten, wobei die Gruppe $R_7$ gegebenenfalls mit einer Hydroxygruppe substituiert ist;

B) U bedeutet -O- oder $-NR_9-$, wobei $R_9$ eine einwertige Gruppe ist, die ausgewählt ist unter einem Wasserstoffatom, einer geradkettigen oder verzweigten Alkylgruppe mit 1 bis 6 Kohlenstoffatomen; einer zweiwertigen Gruppe $-R_1-$ mit den oben angegebenen Bedeutungen, wobei eine Bindung zu dem Stickstoffatom von $-NR_9-$ geht und die andere an ein Siliciumatom gebunden ist;
C) $R_2$ sind Gruppen, die gleich oder verschieden sind und die unter den geradkettigen oder verzweigten Alkylgruppen mit 1 bis 3 Kohlenstoffatomen und Phenyl ausgewählt sind;
D) $R_3$ bedeutet ein Wasserstoffatom oder die Gruppe $R_2$;

■ a ist eine Zahl, die unter 0, 1 und 2 ausgewählt ist;
■ b ist eine Zahl, die unter 0, 1 und 2 ausgewählt ist;
■ die Summe a + b ist höchstens 2.

**2.** Zusammensetzung nach Anspruch 1, **dadurch gekennzeichnet, dass** die reinigenden grenzflächenaktiven Stoffe unter den anionischen grenzflächenaktiven Stoffen ausgewählt sind.

**3.** Zusammensetzung nach Anspruch 1 oder 2, **dadurch gekennzeichnet, dass** das grenzflächenaktive Stoff in einem Gewichtsanteil von 4 bis 50 Gew.-%, bezogen auf das Gesamtgewicht der Zusammensetzung, enthalten ist.

**4.** Zusammensetzung nach Anspruch 3, **dadurch gekennzeichnet, dass** dieser Mengenanteil im Bereich von 6 bis 35 Gew.-%, bezogen auf das Gesamtgewicht der Zusammensetzung, liegt.

**5.** Zusammensetzung nach einem der Ansprüche 1 bis 4, **dadurch gekennzeichnet, dass** das aminierte Silicon ferner mindestens eine weitere Siloxyeinheit der Formel (III) enthält:

$$(R)_c(X)_dVSi(O)_{\frac{3-(c+d)}{2}} \qquad \text{(III)}$$

in der Formel:

■ die Symbole R und X weisen die in Bezug auf die Formel (I) angegebenen Bedeutungen auf:
■ das Symbol V bedeutet: eine geradkettige oder verzweigte Alkylgruppe mit 5 bis 20 Kohlenstoffatomen; eine Gruppe der Formel $-(CH_2)_p-COOR_{12}$, worin p eine Zahl von 5 bis 20 bedeutet und $R_{12}$ eine geradkettige oder verzweigte Alkylgruppe mit 1 bis 12 Kohlenstoffatomen ist; eine Gruppe der Formel $-(CH_2)_q-O-R_{13}$, worin q eine Zahl von 3 bis 10 bedeutet und $R_{13}$ ein Wasserstoffatom, Ethylenoxid, eine mono- oder polyethoxylierte Gruppe, eine mono- oder polypropoxylierte Gruppe, eine gemischte Gruppierung, die aus Ethylenoxideinheiten und Propylenoxideinheiten gebildet ist, oder eine Acylgruppe mit 2 bis 12 Kohlenstoffatomen;

- ■ c ist eine Zahl, die unter 0, 1 und 2 ausgewählt ist;
- ■ d ist eine Zahl, die unter 0, 1 und 2 ausgewählt ist;
- ■ die Summe c + d ist höchstens 2.

**6.** Zusammensetzung nach einem der Ansprüche 1 bis 5, **dadurch gekennzeichnet, dass** das aminierte Silicon ferner (eine) weitere Siloxyeinheit(en) der Formel (IV) enthält:

$$\text{(IV)} \qquad (R)_e(X)_f Si(O)_{\frac{4-(e+f)}{2}}$$

in der Formel:

- ■ die Symbole R und X weisen die in Bezug auf die Formel (I) angegebenen Bedeutungen auf:
- ■ e ist eine Zahl, die unter 0, 1, 2 und 3 ausgewählt ist;
- ■ f ist eine Zahl, die unter 0, 1, 2 und 3 ausgewählt ist;
- ■ die Summe e + f ist höchstens 3.

**7.** Zusammensetzung nach einem der Ansprüche 1 bis 6, **dadurch gekennzeichnet, dass** das aminierte Silicon ein lineares Polyorganosiloxan der mittleren Formel (V) ist:

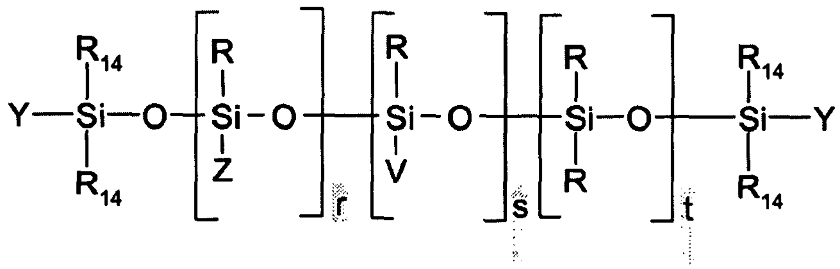

(V)

in der Formel:

- ■ die Symbole R, Z und V weisen die in Bezug auf die Formeln (I) und (III) angegebenen Bedeutungen auf;
- ■ das Symbol Y bedeutet eine einwertige Gruppe, die unter den Gruppen R, Z, V und X ausgewählt ist;
- ■ die Symbole $R_{14}$ sind gleich oder verschieden und bedeuten eine einwertige Gruppe, die unter einer Gruppe R und einer Gruppe X ausgewählt sind, wie sie oben in Bezug auf die Formel (I) definiert sind;
- ■ r, s und t sind Null oder sie bedeuten ganze oder rationale Zahlen über Null, mit der weiteren Maßgabe, dass mindestens eine der beiden Gruppen Y die Gruppe Z bedeutet, wenn r = 0.

**8.** Zusammensetzung nach einem der Ansprüche 1 bis 7, **dadurch gekennzeichnet, dass** das aminierte Silicon ein Polyorganosiloxan mit Einheit(en) der Formeln (I) + gegebenenfalls (III) + gegebenenfalls (IV) oder ein Polyorganosiloxan der Formel (V) bedeutet:
wobei diese Polyorganosiloxane im Mittel pro Mol aufweisen: 2 bis 1600 Siliciumatome, 1 bis 100 wie oben definierte Gruppen Z und 0 bis 50 wie oben definierte Gruppen V;
und in ihrer Struktur:

- R ist unter den Gruppen Methyl, Ethyl, n-Propyl und Isopropyl und vorzugsweise Methyl ausgewählt;
- X ist unter den Gruppen Hydroxy, Methoxy und Ethoxy ausgewählt;
- Z ist unter den Gruppen mit Piperidinylgruppe(n) der Formel (II) ausgewählt, in der:

A) $R_1$ ist eine zweiwertige Kohlenwasserstoffgruppe, die gegebenenfalls ein oder mehrere Heteroatome

wie O und N enthält und die ausgewählt ist unter:

i) linearen oder verzweigten Alkylengruppen mit 2 bis 18 Kohlenstoffatomen;
j) linearen oder verzweigten Alkylengruppen mit 3 bis 12 Kohlenstoffatomen;
k) der Gruppe $-(CH_2)_{10}-CO-$;
l) Alkylen-cyclohexylengruppen, deren linearer oder verzweigter Alkylenteil 2 bis 6 Kohlenstoffatome aufweist und deren Cyclohexylenteil eine OH-Gruppe und gegebenenfalls 1 oder 2 Alkylsubstituenten mit 1 bis 4 Kohlenstoffatomen aufweist;
m) den Gruppen der Formel $-R_4-O-R_5-$, worin die Gruppen $R_4$ und $R_5$, die gleich oder verschieden sind, Alkylengruppen mit 2 bis 6 Kohlenstoffatomen bedeuten;
n) den Gruppen der Formel $-R_4-O-R_5-$, worin die Gruppen $R_4$ und $R_5$ die oben angegebenen Bedeutungen aufweisen, wobei $R_5$ mit einer OH-Gruppe substituiert ist;
o) den Gruppen der Formel $-R_4-COO-R_5-$ und $-R_4-OCO-R_5$, worin die Gruppen $R_4$ und $R_5$ die oben angegebenen Bedeutungen aufweisen;
p) den Gruppen der Formel $-R_6-O-R_7-O-CO-R_8-$, worin die Gruppen $R_6$, $R_7$ und $R_8$, die gleich oder verschieden sind, Alkylengruppen mit 2 bis 6 Kohlenstoffatomen bedeuten und die Gruppe $R_7$ mit einer Hydroxygruppe substituiert ist;

B) U bedeutet -O- oder $-NR_9-$, wobei $R_9$ eine geradkettige oder verzweigte Alkylgruppe mit 1 bis 4 Kohlenstoffatomen ist;
C) $R_2$ bedeutet Methyl;

■ V ist ausgewählt unter: einer geradkettigen oder verzweigten Alkylgruppe mit 5 bis 18 Kohlenstoffatomen; einer Gruppe der Formel $-(CH_2)_{10}-COO-R_{12}$, worin $R_{12}$ eine geradkettige Alkylgruppe mit 1 bis 6 Kohlenstoffatomen ist; einer Gruppe der Formel $-(CH_2)_3-O-R_{13}$, worin $R_{13}$ ein Wasserstoffatom, eine Ethylenoxidgruppe, eine mono- oder polyethoxylierte Gruppe, eine mono- oder polypropoxylierte Gruppe, eine gemischte Gruppierung, die aus Ethylenoxideinheiten und Propylenoxideinheiten gebildet ist, oder eine Acylgruppe mit 2 bis 6 Kohlenstoffatomen ist.

9. Zusammensetzung nach Anspruch 8, **dadurch gekennzeichnet, dass** das aminierte Silicon ein lineares Polyorganosiloxan der Formel (V) ist, das für die Symbole r, s und t die folgenden Werte aufweist:

■ r ist eine ganze oder rationale Zahl im Bereich von 0 bis 98, mit der Maßgabe, dass mindestens eine der beiden Gruppen Y die Gruppe Z bedeutet, wenn r = 0;
■ s ist eine ganze oder rationale Zahl im Bereich von 0 bis 48;
■ t ist eine ganze oder rationale Zahl im Bereich von 0 bis 1598;
■ die Summe r + s + t ist eine ganze oder rationale Zahl im Bereich von 0 bis 1598.

10. Zusammensetzung nach einem der Ansprüche 1 bis 9, **dadurch gekennzeichnet, dass** das aminierte Silicon ein Polyorganosiloxan mit der folgenden Struktur ist:

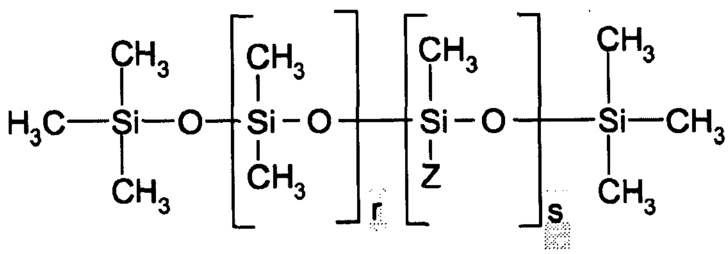

mit Z =

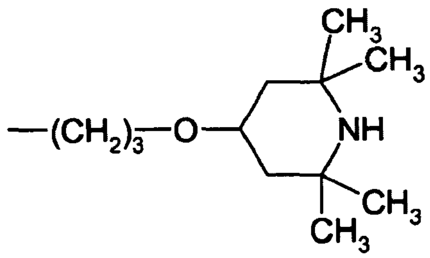

s liegt im Bereich von 1 bis 50 und vorzugsweise 2 bis 30;
r liegt im Bereich von 5 bis 2000 und vorzugsweise 500 bis 1700.

**11.** Zusammensetzung nach einem der Ansprüche 1 bis 10, **dadurch gekennzeichnet, dass** das aminierte Silicon in Konzentrationen von 0,001 bis 10 Gew.-%, vorzugsweise 0,01 bis 5 Gew.-% und noch bevorzugter 0,1 bis 3 Gew.-%, bezogen auf das Gesamtgewicht der fertigen Zusammensetzung, enthalten ist.

**12.** Zusammensetzung nach einem der Ansprüche 1 bis 11, **dadurch gekennzeichnet, dass** das kationische Polymer ausgewählt ist unter:

(1) Homopolymeren oder Copolymeren, die von Acrylsäureestern, Methacrylsäureestern, Acrylamiden oder Methacrylamiden abgeleitet sind und mindestens eine der folgenden Einheiten enthalten:

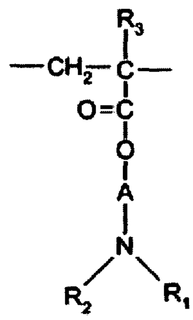
,
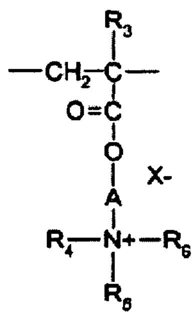

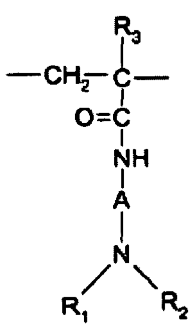

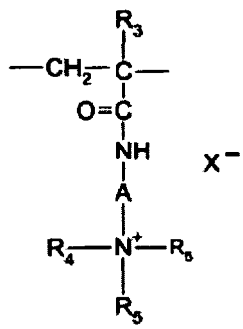

in den Formeln:

die Gruppen $R_3$, die gleich oder verschieden sind, bedeuten ein Wasserstoffatom oder die Gruppe $CH_3$;
die Gruppen A, die gleich oder verschieden sind, bedeuten eine lineare oder verzweigte Alkylgruppe mit 1 bis 6 Kohlenstoffatomen und vorzugsweise 2 oder 3 Kohlenstoffatomen oder eine Hydroxyalkylgruppe mit 1 bis 4 Kohlenstoffatomen;
die Gruppen $R_4$, $R_5$ und $R_6$, die gleich oder verschieden sind, bedeuten eine Alkylgruppe mit 1 bis 18

Kohlenstoffatomen oder eine Benzylgruppe und vorzugsweise eine Alkylgruppe mit 1 bis 6 Kohlenstoffatomen;

die Gruppen $R_1$ und $R_2$, die gleich oder verschieden sind, bedeuten Wasserstoff oder eine Alkylgruppe mit 1 bis 6 Kohlenstoffatomen und vorzugsweise Methyl oder Ethyl;

die Gruppe X bedeutet ein Anion, das von einer anorganischen oder organischen Säure abgeleitet ist, wie ein Methosulfatanion oder ein Halogenid wie Chlorid oder Bromid;

(2) Kationischen Polysacchariden;

(3) Polymeren, die aus Piperazinyleinheiten und zweiwertigen Alkylen- oder Hydroxyalkylengruppen mit geraden oder verzweigten Ketten bestehen, die gegebenenfalls durch Sauerstoffatome, Schwefelatome, Stickstoffatome oder aromatische oder heterocyclische Ringe unterbrochen sind, sowie die Oxidationsprodukte und/oder Quaternisierungsprodukte dieser Polymere;

(4) Wasserlöslichen Polyaminoamiden, die insbesondere durch Polykondensation einer sauren Verbindung mit einem Polyamin hergestellt sind;

(5) Derivaten von Polyaminoamiden, die bei der Kondensation von Polyalkylenpolyaminen mit Polycarbonsäuren und anschließender Alkylierung mit bifunktionellen Mitteln entstehen;

(6) Polymeren, die durch Umsetzung eines Polyalkylenpolyamins, das zwei primäre Aminogruppen und mindestens eine sekundäre Aminogruppe aufweist, mit einer Dicarbonsäure hergestellt sind;

(7) Alkyldiallylamin- oder Dialkyldiallylammonium-Cyclopolymeren;

(8) Quartären Diammoniumpolymeren mit Wiederholungseinheiten der folgenden Formel:

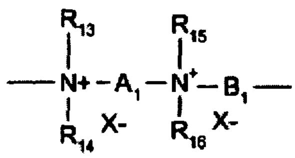

(II)

in der Formel (II):

die Gruppen $R_{13}$, $R_{14}$, $R_{15}$ und $R_{16}$, die identisch oder voneinander verschieden sind, bedeuten aliphatische, alicyclische oder arylaliphatische Gruppen mit 1 bis 20 Kohlenstoffatomen oder niedere hydroxyalkylaliphatische Gruppen oder die Gruppen $R_{13}$, $R_{14}$, $R_{15}$ und $R_{16}$ bilden gemeinsam oder getrennt voneinander mit den Stickstoffatomen, an die sie gebunden sind, Heterocyclen, die gegebenenfalls ein von Stickstoff verschiedenes, zweites Heteroatom enthalten, oder die Gruppen $R_{13}$, $R_{14}$, $R_{15}$ und $R_{16}$ bedeuten eine geradkettige oder verzweigte $C_{1-6}$-Alkylgruppe, die mit einer Nitrilgruppe, Estergruppe, Acylgruppe, Amidgruppe oder -CO-O-$R_{17}$-D oder -CO-NH-$R_{17}$-D substituiert ist, wobei $R_{17}$ eine Alkylengruppe bedeutet und D eine quartäre Ammoniumgruppe ist;

die Gruppen $A_1$ und $B_1$ bedeuten Polymethylengruppen, die 2 bis 20 Kohlenstoffatome aufweisen und linear oder verzweigt, gesättigt oder ungesättigt sein können und an die Hauptkette gebunden oder eingebaut in die Hauptkette einen oder mehrere aromatische Ringe oder ein oder mehrere Sauerstoffatome, Schwefelatome oder eine oder mehrere der folgenden Gruppen enthalten können: Sulfoxid, Sulfon, Disulfid, Amino, Alkylamino, Hydroxy, quartäres Ammonium, Ureido, Amid oder Ester, und

$X^-$ bedeutet ein Anion, das von einer anorganischen oder organischen Säure abgeleitet ist;

$A_1$, $R_{13}$ und $R_{15}$ können mit den beiden Stickstoffatomen, an die sie gebunden sind, einen Piperazinring bilden; wenn $A_1$ eine lineare oder verzweigte, gesättigte oder ungesättigte Alkylen- oder Hydroxyalkylengruppe ist, kann $B_1$ auch eine Gruppe $(CH_2)_n$-CO-D-OC-$(CH_2)_n$- bedeuten,

worin D bedeutet:

(a) einen Glycolrest der Formel: -O-Z-O-, wobei Z eine lineare oder verzweigte Kohlenwasserstoffgruppe ist oder eine Gruppe bedeutet, die einer der folgenden Formeln entspricht:

(CH2-CH2-O)x-CH2-CH2-

-[CH2-CH(CH3)-O]y-CH2-CH(CH3)-

wobei x und y eine ganze Zahl von 1 bis 4 bedeuten, die einen einzigen und wohldefinierten Polymerisationsgrad bedeutet, oder eine beliebige Zahl von 1 bis 4, die einen mittleren Polymerisationsgrad angibt;
(b) einen bis-sekundären Diaminrest, beispielsweise ein Piperazinderivat;
(c) einen bis-primären Diaminrest der Formel: -NH-Y-NH-, wobei Y eine lineare oder verzweigte Kohlenwasserstoffgruppe ist, oder die zweiwertige Gruppe

$$-CH_2-CH_2-S-S-CH_2-CH_2-;$$

(d) eine Ureylengruppe der Formel: -NH-CO-NH;

$X^-$ ist ein Anion;

(9) quartären Polyammoniumpolymeren, die Einheiten der folgenden Formel (III) enthalten:

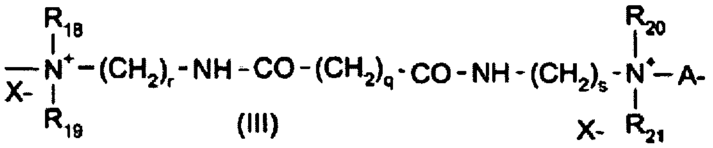

(III)

in der Formel:

$R_{18}$, $R_{19}$, $R_{20}$ und $R_{21}$, die identisch oder verschieden sind, bedeuten ein Wasserstoffatom oder eine Gruppe Methyl, Ethyl, Propyl, β-Hydroxyethyl, β-Hydroxypropyl oder $-CH_2CH_2(OCH_2CH_2)_pOH$,
wobei p 0 ist oder eine ganze Zahl von 1 bis 6 bedeutet, mit der Maßgabe, dass die Gruppen $R_{18}$, $R_{19}$, $R_{20}$ und $R_{21}$ nicht gleichzeitig ein Wasserstoffatom bedeuten,
r und s, die gleich oder verschieden sind, sind ganze Zahlen im Bereich von 1 bis 6,
q bedeutet 0 oder eine ganze Zahl von 1 bis 34,
X ist ein Anion, beispielsweise ein Halogenid,
A bedeutet eine Dihalogenidgruppe oder vorzugsweise $-CH_2-CH_2-O-CH_2-CH_2-$;

(10) quartären Polymeren von Vinylpyrrolidon und Vinylimidazol;
(11) Vernetzten Polymeren von Methacryloyloxyalkyl($C_{1-4}$)trialkyl($C_{1-4}$)ammoniumsalzen;
(12) Kationischen Proteinen oder kationischen Proteinhydrolysaten, Polyalkyleniminen, insbesondere Polyethyleniminen, Polymeren, die Vinylpyridin- oder Vinylpyridinium-Einheiten enthalten, Kondensaten von Polyaminen und Epichlorhydrin, quartären Polyureylenen und Chitinderivaten.

**13.** Zusammensetzung nach Anspruch 12, **dadurch gekennzeichnet, dass** das kationische Polymer unter den quartären Celluloseetherderivaten, Homopolymeren von Diallyldimethylammoniumsalzen, Copolymeren von Diallyldimethylammoniumsalzen und Acrylamid, kationischen Polysacchariden, die nicht auf Cellulose basieren, quartären Copolymeren von Vinylpyrrolidon und einem Vinylimidazolsalz ausgewählt ist.

**14.** Zusammensetzung nach einem der Ansprüche 11 bis 13, **dadurch gekennzeichnet, dass** das oder die kationische (n) Polymer(e) 0,001 bis 20 Gew.-% und vorzugsweise 0,01 bis 10 Gew.-%, bezogen auf das Gesamtgewicht der fertigen Zusammensetzung, ausmachen.

**15.** Zusammensetzung nach einem der vorhergehenden Ansprüche, **dadurch gekennzeichnet, dass** die Zusammensetzung ferner mindestens ein Silicon enthält, das von den aminierten Siliconen verschieden ist, die eine von einer sterisch gehinderten Gruppe getragene Aminofunktion aufweist.

**16.** Zusammensetzung nach dem vorhergehenden Anspruch, **dadurch gekennzeichnet, dass** das von den aminierten Siliconen verschiedene Silicon in einer Menge von 0,01 bis 20 Gew.-% und vorzugsweise 0, 1 bis 5 Gew.-%, bezogen auf das Gesamtgewicht der Zusammensetzung, verwendet wird.

**17.** Zusammensetzung nach einem der vorhergehenden Ansprüche, **dadurch gekennzeichnet, dass** sie einen pH-Wert im Bereich von 3 bis 10 aufweist.

18. Zusammensetzung nach einem der Ansprüche 1 bis 17, **dadurch gekennzeichnet, dass** sie ferner einen oder mehrere Zusatzstoffe enthält, die unter den kationischen grenzflächenaktiven Stoffen, anionischen oder nichtionischen oder amphoteren Polymeren, Proteinen, Proteinhydrolysaten, Ceramiden, Pseudoceramiden, pflanzlichen Ölen, Mineralölen oder synthetischen Ölen, Fettsäuren, Hydroxysäuren, Vitaminen, Provitaminen wie Panthenol, UV-Filtern, Hydratisierungsmitteln, Antischuppenmitteln, Wirkstoffen gegen Seborrhoe, Wirkstoffen gegen Haarausfall, Radikalfängern für freie Radikale, Trübungsmitteln und deren Gemischen ausgewählt sind.

19. Verwendung einer Zusammensetzung nach einem der vorhergehenden Ansprüche in der Kosmetik für die Reinigung und/oder für die Pflege und/oder zum Konditionieren und/oder zum Frisieren der Haare.

20. Verwendung einer Zusammensetzung nach einem der Ansprüche 1 bis 18 für die Reinigung und/ oder zum Abschminken von Keratinsubstanzen.

21. Verfahren für die Reinigung und Pflege von Keratinsubstanzen, wie Haaren, das darin besteht, eine wirksame Menge einer Zusammensetzung nach einem der Ansprüche 1 bis 18 auf die feuchten Keratinsubstanzen aufzutragen und anschließend nach einer gegebenenfalls abgewarteten Einwirkzeit mit Wasser zu spülen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2003130760 A
- EP 0739625 A
- EP 0974335 A
- WO 9746211 A
- US 2528378 A
- US 2781354 A
- EP 0337354 A
- FR 2270846 A
- FR 2383660 A
- FR 2598611 A
- FR 2470596 A
- FR 2519863 A
- FR 2505348
- FR 2542997
- EP 080976 A
- FR 2077143
- FR 2393573
- FR 1492597
- US 4131576 A
- US 3589578 A
- US 4031307 A
- FR 2162025
- FR 2280361
- FR 2252840
- FR 2368508
- FR 1583363
- FR 2080759
- FR 2320330
- FR 2270846
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- FR 1400366
- FR 8516334 A
- US 4957732 A
- EP 186507 A
- EP 342834 A


### Littérature non-brevet citée dans la description


- **M.R. PORTER.** Handbook of Surfactants. 1991, 116-178
- CTFA. 1991
- **WALTER NOLL.** Chemistry and Technology of Silicones. Academie Press, 1968
- **TODD ; BYERS.** Volatile Silicone fluids for cosmetics. *Cosmetics and toiletries,* Janvier 1976, vol. 91, 27-32